# EUROPEAN PATENT SPECIFICATION

(11) **EP 4 347 565 B1**
(45) Date of publication and mention of the grant of the patent: **02.04.2025**
(21) Application number: 22732474.6
(22) Date of filing: 01.06.2022
(51) Int. Cl.: C07D 211/38, C07D 211/52, C07D 401/10, C07D 401/14

(54) **PREPARATION OF A CHK1 INHIBITOR COMPOUND**
HERSTELLUNG EINER CHK1-INHIBITORVERBINDUNG
PRÉPARATION D'UN COMPOSÉ INHIBITEUR DE CHK1

(30) Priority: 03.06.2021 GB 202107932
(43) Date of publication of application: 10.04.2024
(60) Divisional application: 25158535.2
(73) Proprietor: Sentinel Oncology Limited, Cambridge Cambridgeshire CB4 0GJ (GB); Pharmaengine, Inc., Taiwan 10480 (TW)
(72) Inventor: TRAVERS, Stuart, Cambridge Cambridgeshire CB4 0GJ (GB); MAJOR, Meriel, Cambridge Cambridgeshire CB4 0GJ (GB); LONDESBROUGH, Derek John, Sunderland Tyne and Wear SR5 2TQ (GB); CHUBB, Richard, Sunderland Tyne and Wear SR5 2TQ (GB)
(74) Representative: Schlich
(86) International application number: PCT/EP2022/064903
(87) International publication number: WO 2022/253895

(56) References cited:
- WO-A1-2015/120390

## Description

This invention relates to processes for preparing the Chk-1 inhibiitor compound 5-[[5-[4-(4-fluoro-1-methyl-4-piperidyl)-2-methoxy-phenyl]-1H-pyrazol-3-yl]amino]pyrazine-2-carbonitrile, processes for preparing synthetic intermediates, and to novel chemical intermediates for use in the processes.

### Background of the Invention

Chk-1 is a serine/threonine kinase involved in the induction of cell cycle checkpoints in response to DNA damage and replicative stress [Tse et al, Clin. Can. Res. 2007;13(7)]. Cell cycle checkpoints are regulatory pathways that control the order and timing of cell cycle transitions. Many cancer cells have impaired G1 checkpoint activation. For example, Hahn *et al.,* and Hollstein *et al.,* have reported that tumours are associated with mutations in the p53 gene, a tumour suppressor gene found in about 50% of all human cancers [N Engl J Med 2002, 347(20):1593; Science, 1991, 253(5015):49].

Chk-1 inhibition abrogates the intra S and G2/M checkpoints and has been shown to selectively sensitise tumour cells to well known DNA damaging agents. Examples of DNA damaging agents where this sensitising effect has been demonstrated include Gemcitabine, Pemetrexed, Cytarabine, Irinotecan, Camptothecin, Cisplatin, Carboplatin [Clin. Cancer Res. 2010, 16, 376], Temozolomide [Journal of Neurosurgery 2004, 100, 1060], Doxorubicin [Bioorg. Med. Chem. Lett. 2006;16:421-6], Paclitaxel [WO2010149394], Hydroxy urea [Nat. Cell. Biol. 2005;7(2):195-20], the nitroimidazole hypoxia-targetted drug TH-302 (Meng et al., AACR, 2013 Abstract No. 2389) and ionising radiation [Clin. Cancer Res. 2010, 16, 2076]. See also the review article by McNeely et al., [Pharmacology & Therapeutics (2014), 142(1):1-10]

Recently published data have also shown that Chk-1 inhibitors may act synergistically with PARP inhibitors [Cancer Res 2006.; 66:(16)], Mek inhibitors [Blood. 2008; 112(6): 2439-2449], Farnesyltransferase inhibitors [Blood. 2005;105(4):1706-16], Rapamycin [Mol. Cancer Ther. 2005;4(3):457-70], Src inhibitors [Blood. 2011;117(6):1947-57] and WEE1 inhibitors [Carrassa, 2021, 11(13):2507; Chaudhuri et al., Haematologica, 2014 99(4):688.].

Furthermore, Chk-1 inhibitors have demonstrated an advantage when combined with immunotherapy agents [Mouw et al., Br J Cancer, 2018. (7):933]. Chk1 inhibitors have been shown to activate cGAS, which induces an innate immune response through STING signaling, and to induce PD-L1 expression and synergize with anti-PD-L1 in vivo [Sen et al., Cancer Discov 2019 (5):646; Sen et al., J Thorac Oncol, 2019. (12):2152].

Resistance to chemotherapy and radiotherapy, a clinical problem for conventional therapy, has been associated with activation of the DNA damage response in which Chk-1 has been implicated [Nature; 2006; 444(7):756-760; Biochem. Biophys. Res. Commun. 2011 ;406(1):53-8].

It is also envisaged that Chk-1 inhibitors, either as single agents or in combination, may be useful in treating tumour cells in which constitutive activation of DNA damage and checkpoint pathways drive genomic instability in particular through replication stress. This phenotype is associated with complex karyotypes, for example in samples from patients with acute myeloid leukemia (AML) [Cancer Research 2009, 89, 8652]. *In vitro* antagonisation of the Chk-1 kinase with a small molecule inhibitor or by RNA interference strongly reduces the clonogenic properties of high-DNA damage level AML samples. In contrast Chk-1 inhibition has no effect on normal hematopoietic progenitors. Furthermore, recent studies have shown that the tumour microenvironment drives genetic instability [Nature; 2008;(8):180-192] and loss of Chk-1 sensitises cells to hypoxia/reoxygenation [Cell Cycle; 2010; 9(13):2502]. In neuroblastoma, a kinome RNA interference screen demonstrated that loss of Chk-1 inhibited the growth of eight neuroblastoma cell lines. Tumour cells deficient in Fanconi anemia DNA repair have shown sensitivity to Chk-1 inhibition [Molecular Cancer 2009, 8:24]. It has been shown that the Chk-1 specific inhibitor PF-00477736 inhibits the growth of thirty ovarian cancer cell lines [Bukczynska et al, 23rd Lorne Cancer Conference] and triple negative breast cancer cells [Cancer Science 2011, 102, 882]. Also, PF-00477736 has displayed selective single agent activity in a MYC oncogene driven murine spontaneous cancer model [Ferrao et al, Oncogene (15 August 2011)]. Chk-1 inhibition, by either RNA interference or selective small molecule inhibitors, results in apoptosis of MYC-overexpressing cells both *in vitro* and in an *in vivo* mouse model of B-cell lymphoma [Höglund et al., Clinical Cancer Research, 2011]. The latter data suggest that Chk-1 inhibitors would have utility for the treatment of MYC-driven malignancies such as B-cell lymphoma/leukemia, neuroblastoma and some breast and lung cancers. Chk-1 inhibitors have also been shown to be effective in pediatric tumour models, including Ewing's sarcoma and rhabdomyosarcoma [Lowery, 2018. Clin Cancer Res 2019, 25(7):2278]. Chk1 inhibitors have been shown to be synthetically lethal with the B-family of DNA polymerases, resulting in increased replication stress, DNA damage and cell death [Rogers et al., 2020, 80(8);1735]. Other cell cycle regulated genes have also been reported to confer sensitivity to Chk-1 inhibitors, including CDK2 and POXM1 [Ditano et al., 20201. 11(1);7077; Branigan et al., 2021 Cell Reports 34(9):1098808]

It has also been reported that mutations that reduce the activity of DNA repair pathways can result in synthetically lethal interactions with Chk1 inhibition. For example, mutations that disrupt the RAD50 complex and ATM signaling increase responsiveness to Chk1 inhibition [Al-Ahmadie et al., Cancer Discov. 2014. (9):1014-21]. Likewise, deficiencies in the Fanconi anemia homologous DNA repair pathway lead to sensitivity to Chk1 inhibition [Chen et al.,. Mol. Cancer 2009 8:24, Duan et al., Frontiers in Oncology 2014 4:368]. Also, human cells that have loss of function in the Rad17 gene product are sensitive to Chk1 suppression [Shen et al., Oncotarget, 2015. 6(34):35755].

Various attempts have been made to develop inhibitors of Chk-1 kinase. For example, WO 03/10444 and WO 2005/072733 (both in the name of Millennium) disclose aryl/heteroaryl urea compounds as Chk-1 kinase inhibitors. US2005/215556 (Abbott) discloses macrocyclic ureas as kinase inhibitors. WO 02/070494, WO2006014359 and WO2006021002 (all in the name of Icos) disclose aryl and heteroaryl ureas as Chk-1 inhibitors. WO/2011/141716 and WO/2013/072502 both disclose substituted pyrazinyl-phenyl ureas as Chk-1 kinase inhibitors. WO2005/009435 (Pfizer) and WO2010/077758 (Eli Lilly) disclose aminopyrazoles as Chk-1 kinase inhibitors.

WO2015/120390 discloses a class of substituted phenyl-pyrazolyl-amines as Chk-1 kinase inhibitors. One of the compounds disclosed is the compound 5-[[5-[4-(4-fluoro-1-methyl-4-piperidyl)-2-methoxy-phenyl]-1H-pyrazol-3-yl]amino]pyrazine-2-carbonitrile, the synthesis of which is described in Example 64 and Synthetic Method L in WO2015/120390.

The Chk-1 kinase inhibitor compound 5-[[5-[4-(4-fluoro-1-methyl-4-piperidyl)-2-methoxy-phenyl]-1H-pyrazol-3-yl]amino]pyrazine-2-carbonitrile is useful in the treatment of cancers as disclosed in WO2015/120390.

WO2018/183891 (Cascadian Therapeutics) discloses combinations of the compound 5-[[5-[4-(4-fluoro-1-methyl-4-piperidyl)-2-methoxy-phenyl]-1H-pyrazol-3-yl]amino]pyrazine-2-carbonitrile or a pharmaceutically acceptable salt thereof with WEE-1 inhibitors.

### The Invention

The present invention provides improved processes for making the Chk-1 kinase inhibitor compound 5-[[5-[4-(4-fluoro-1-methyl-4-piperidyl)-2-methoxy-phenyl]-1H-pyrazol-3-yl]amino]pyrazine-2-carbonitrile (referred to herein also as the compound of formula (I) or the Chk-1 inhibitor).

The improved process is represented by the sequence of reactions set out in Scheme 1 below.

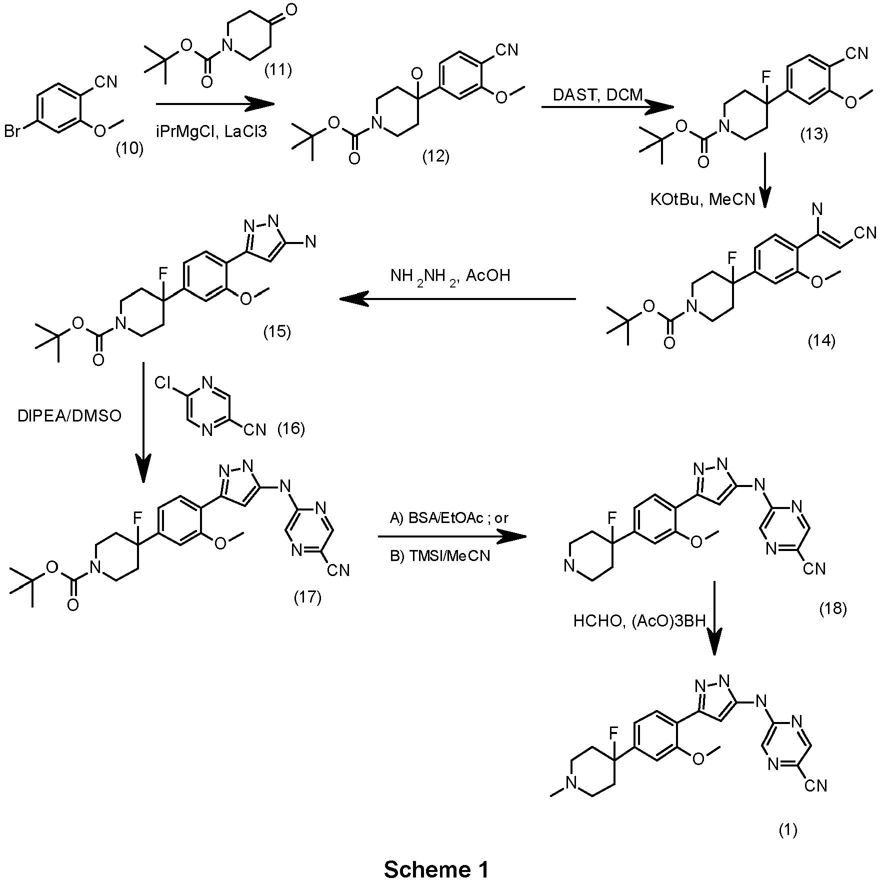

The synthetic route shown in Scheme 1 has a number of advantages over the synthetic route described in WO2015/120390. For example, the route depicted in Scheme 1 is shorter in terms of the total steps (7 vs 9). Many of the intermediates derived from the process are readily isolable crystalline solids. The new route therefore also makes use of these crystalline intermediates to remove the need for chromatography and thereby is a more scalable process. In addition, the improved process avoids the use of certain reagents in WO2015/120390 which are undesirable for large scale synthesis (e.g. Dess-Martin periodinane and n-BuLi).

The improved synthetic route makes use of the same final step (reductive methylation) as the synthetic route described in WO 2015/120390 but the deprotection and synthesis of the Boc-protected intermediate of formula (17) in the present route differ from the synthesis and deprotection of intermediate of formula (17) in WO2015/120390.

In one aspect (Embodiment 1.1), the invention provides a process for the preparation of a compound of formula (15): 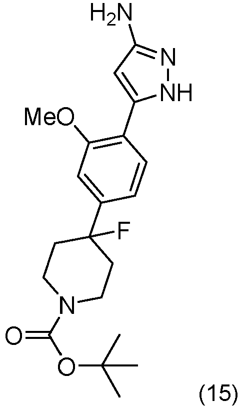 which process comprises reacting a compound of the formula (14): 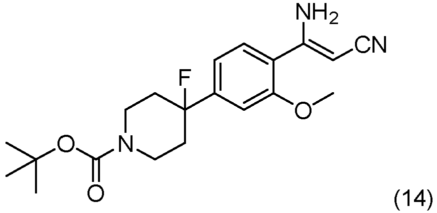 with hydrazine (NH₂NH₂).

This process results in an improved yield compared to the corresponding process for the formation of a compound of formula (15) in WO 2015/120390 (see Example 64). The improved process results in a yield of 58% compared to a yield of 44% obtained with the process of Example 64.

In further embodiments, the invention provides:
1.2 A process according to Embodiment 1.1 wherein the reaction is carried out in a polar, protic solvent.
1.3 A process according to Embodiment 1.2 wherein the polar, protic solvent is a C₁₋₄ alcohol, such as ethanol (EtOH).
1.4 A process according to any one of Embodiments 1.1 to 1.3 wherein the reaction is carried out in the present of glacial acetic acid.
1.5 A process according to any one of Embodiment 1.1 to 1.4 wherein the reaction is carried out at a temperature of 70 °C to 80 °C, for example at a temperature of approximately 75 °C.
1.6 A process according to any one of Embodiments 1.1 to 1.5 wherein the reaction is carried out for a time period of 4 hours or less, for example from 1 hour to 3 hours, such as for approximately 2 hours.

In the improved process, the method of preparing intermediate of formula (15) is entirely different to the method used in WO 2015/120390. The improved method results in a number of advantages described below, including:
- The use of crystalline intermediates (which reduces the need for chromatographic purification of intermediates)
- The lowest temperature used is -10 °C, making the process more suitable for scaling (the route described in WO 2015/120390 involved two reaction steps that were conducted at -78 °C)
- The reactions being higher yielding
- Avoiding the use of Dess-Martin periodinane as an oxidizing reagent. Use of this reagent on an industrial scale is made difficult by its cost and its potentially explosive nature (see Plumb, J.B.; Harper, D.J. (1990). "Chemical Safety: 2-lodoxybenzoic acid". Chem. Eng. News. 68: 3. doi:10.1021/cen-v068n029.p002)

The above processes can be used in the preparation of 5-[[5-[4-(4-fluoro-1-methyl-4-piperidyl)-2-methoxy-phenyl]-1H-pyrazol-3-yl]amino]pyrazine-2-carbonitrile or pharmaceutically acceptable salts thereof. Accordingly, in a further aspect, there is provided a process for the preparation of 5-[[5-[4-(4-fluoro-1-methyl-4-piperidyl)-2-methoxy-phenyl]-1H-pyrazol-3-yl]amino]pyrazine-2-carbonitrile, which process comprises:
i) a process according to Embodiment 1.1; and
ii) interconverting a product obtained from step i) into 5-[[5-[4-(4-fluoro-1-methyl-4-piperidyl)-2-methoxy-phenyl]-1H-pyrazol-3-yl]amino]pyrazine-2-carbonitrile; and
iii) optionally forming a pharmaceutically acceptable salt of 5-[[5-[4-(4-fluoro-1-methyl-4-piperidyl)-2-methoxy-phenyl]-1H-pyrazol-3-yl]amino]pyrazine-2-carbonitrile.

Alternatively, the invention provides a process for the preparation of 5-[[5-[4-(4-fluoro-1-methyl-4-piperidyl)-2-methoxy-phenyl]-1H-pyrazol-3-yl]amino]pyrazine-2-carbonitrile, which process comprises:
i) a process according to Embodiment 1.1;
ii) interconverting a product obtained from step i) to a compound of formula (18); 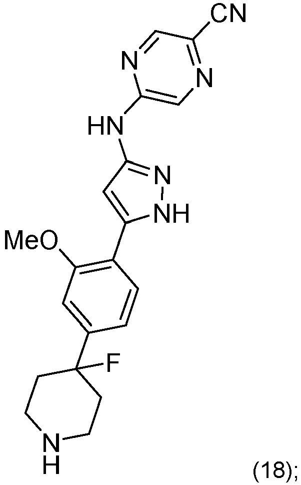
iii) interconverting a product obtained from step ii) into 5-[[5-[4-(4-fluoro-1-methyl-4-piperidyl)-2-methoxy-phenyl]-1H-pyrazol-3-yl]amino]pyrazine-2-carbonitrile, for example by reacting a compound of formula (18) with a methylating agent (such as HCHO in the presence of a reducing agentsuch as (AcO₃)BH); and
iv) optionally forming a pharmaceutically acceptable salt of 5-[[5-[4-(4-fluoro-1-methyl-4-piperidyl)-2-methoxy-phenyl]-1H-pyrazol-3-yl]amino]pyrazine-2-carbonitrile.

In a further aspect, the invention provides novel intermediates suitable for use in the processes of the invention. It has been found that the intermediates are crystalline and therefore the need for chromatography to purify reaction products is reduced or avoided.

Accordingly, in further embodiments, the invention provides:
2.1 A compound of the formula (14): 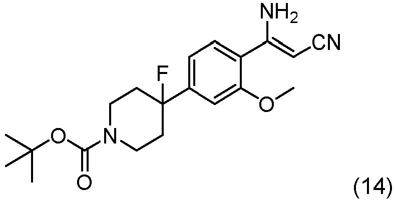
2.2 A compound of the formula (13): 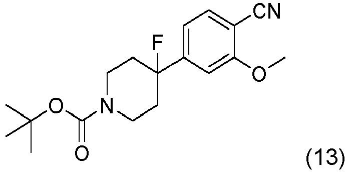
2.3 A compound of the formula (12): 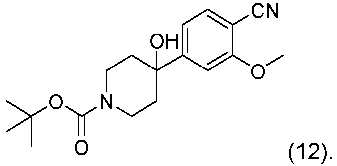
2.4 A compound according to any one of Embodiments 2.1 to 2.3 in a substantially crystalline form.

Further details of crystalline forms of the intermediates used in the improved process are provided below.

### Intermediate of Formula (12)

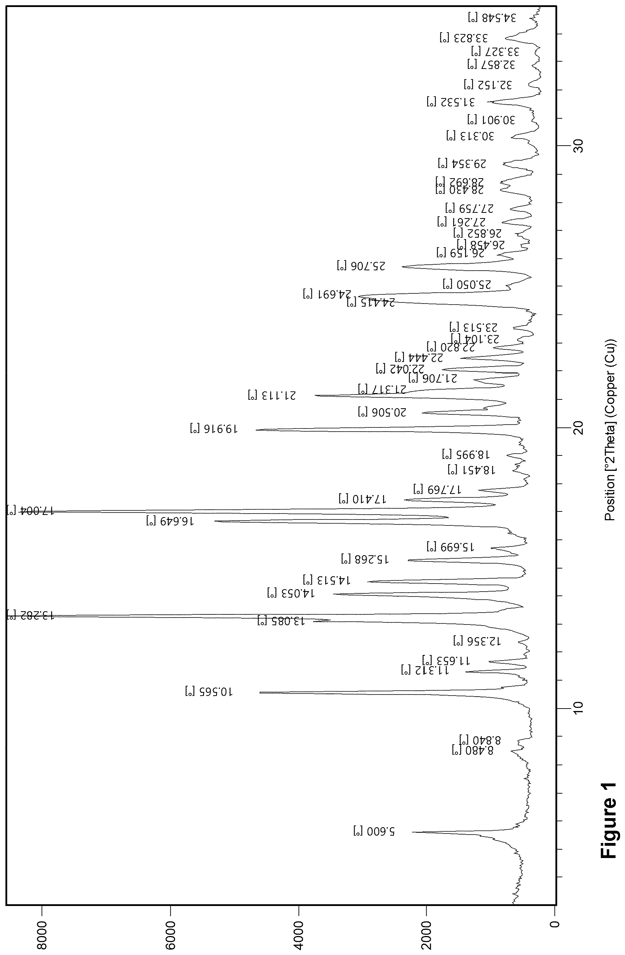

The XRPD diffractogram for a crystalline form of the intermediate of formula (12) is shown in Figure 1.

The X-ray diffraction pattern of the crystalline form of the intermediate of formula (12) exhibits peaks of greatest intensity at the diffraction angles (2θ) set out in Table A-1

| Table A-1 | |
|---|---|
| **Diffraction Angle (°)** | **Relative Intensity** |
| 10.6 ± 0.2 | 54 |
| 13.3 ± 0.2 | 100 |
| 16.7 ± 0.2 | 61 |
| 17.0 ± 0.2 | 99 |
| 19.9 ± 0.2 | 52 |

Accordingly, in further embodiments, the invention provides:
2.5 A substantially crystalline form of the compound of formula (12) having an X-ray powder diffraction pattern characterised by the presence of major peaks at the diffraction angles (2θ) 10.6° and/or 13.3° and/or 16.7° and/or 17.0° and/or 19.9° (±0.2°).
2.6 A substantially crystalline form of the compound of formula (12) having an X-ray powder diffraction pattern characterised by the presence of a major peak at the diffraction angle (2θ) 10.6° (±0.2°).
2.7 A substantially crystalline form of the compound of formula (12) having an X-ray powder diffraction pattern characterised by the presence of a major peak at the diffraction angle (2θ) 13.3° (±0.2°).
2.8 A substantially crystalline form of the compound of formula (12) having an X-ray powder diffraction pattern characterised by the presence of a major peak at the diffraction angle (2θ) 16.7° (±0.2°).
2.9 A substantially crystalline form of the compound of formula (12) having an X-ray powder diffraction pattern characterised by the presence of a major peak at the diffraction angle (2θ) 17.0° (±0.2°).
2.10 A substantially crystalline form of the compound of formula (12) having an X-ray powder diffraction pattern characterised by the presence of a major peak at the diffraction angle (2θ) 19.9° (±0.2°).
2.11 A substantially crystalline form of the compound of formula (12) having an X-ray powder diffraction pattern characterised by the presence of major peaks at two or more, e.g. three or more, or four or more, and in particular five diffraction angles (2θ) selected from 10.6°, 13.3°, 16.7°, 17.0° and 19.9° (±0.2°).
2.12 A substantially crystalline form of the compound of formula (12) which exhibits peaks at the diffraction angles set forth in Table A-1 provided above or Table 1 provided below which have a relative intensity of at least 15%.
2.13 A substantially crystalline form of the compound of formula (12) which exhibits peaks at the diffraction angles corresponding to those of the X-ray powder diffraction pattern shown in Figure 1.
2.14 A substantially crystalline form of the compound of formula (12) having an X-ray powder diffraction pattern substantially as shown in Figure 1.

### Intermediate of Formula (13)

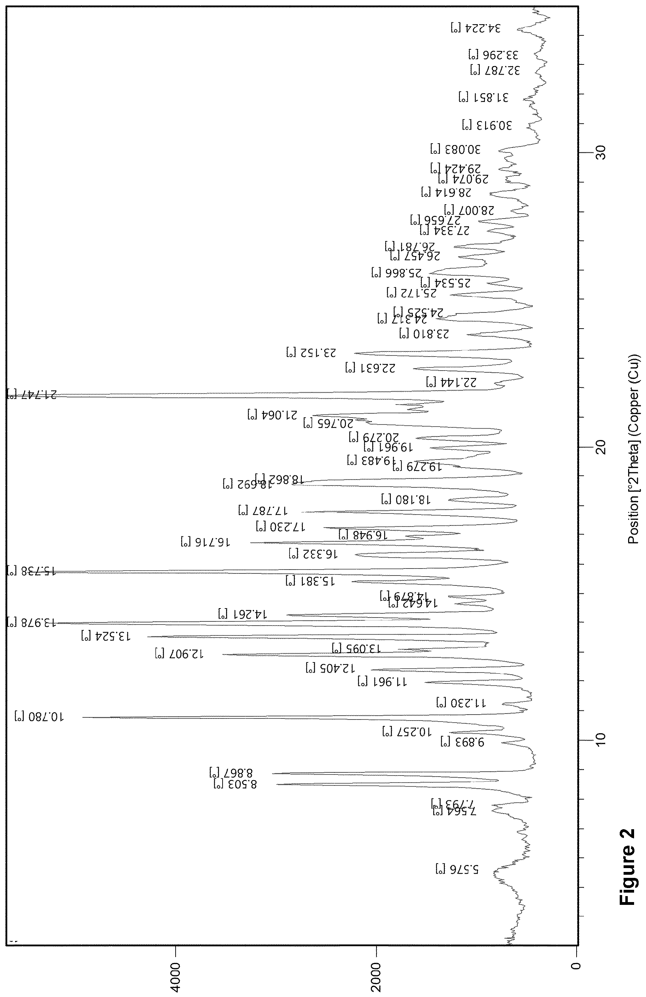

The XRPD diffractogram for a crystalline form of the intermediate of formula (13) is shown in Figure 2.

The X-ray diffraction pattern of the crystalline form of the intermediate of formula (13) exhibits peaks of greatest intensity at the diffraction angles (2θ) set out in Table A-2

| Table A-2 | |
|---|---|
| **Diffraction Angle (°)** | **Relative Intensity** |
| 10.8 ± 0.2 | 87 |
| 13.5 ± 0.2 | 71 |
| 14.0 ± 0.2 | 89 |
| 15.7 ± 0.2 | 91 |
| 21.7 ± 0.2 | 100 |

Accordingly, in further embodiments, the invention provides:
2.15 A substantially crystalline form of the compound of formula (13) having an X-ray powder diffraction pattern characterised by the presence of major peaks at the diffraction angles (2θ) 10.8° and/or 13.5° and/or 14.0° and/or 15.7° and/or 21.7° (±0.2°).
2.16 A substantially crystalline form of the compound of formula (13) having an X-ray powder diffraction pattern characterised by the presence of a major peak at the diffraction angle (2θ) 10.8° (±0.2°).
2.17 A substantially crystalline form of the compound of formula (13) having an X-ray powder diffraction pattern characterised by the presence of a major peak at the diffraction angle (2θ) 13.5° (±0.2°).
2.18 A substantially crystalline form of the compound of formula (13) having an X-ray powder diffraction pattern characterised by the presence of a major peak at the diffraction angle (2θ) 14.0° (±0.2°).
2.19 A substantially crystalline form of the compound of formula (13) having an X-ray powder diffraction pattern characterised by the presence of a major peak at the diffraction angle (2θ) 15.7° (±0.2°).
2.20 A substantially crystalline form of the compound of formula (13) having an X-ray powder diffraction pattern characterised by the presence of a major peak at the diffraction angle (2θ) 21.7° (±0.2°).
2.21 A substantially crystalline form of the compound of formula (13) having an X-ray powder diffraction pattern characterised by the presence of major peaks at two or more, e.g. three or more, or four or more, and in particular five diffraction angles (2θ) selected from 10.8°, 13.5°, 14.0°, 15.7° and 21.7° (±0.2°).
2.22 A substantially crystalline form of the compound of formula (13) which exhibits peaks at the diffraction angles set forth in Table A-2 provided above or Table 2 provided below which have a relative intensity of at least 15%.
2.23 A substantially crystalline form of the compound of formula (13) which exhibits peaks at the diffraction angles corresponding to those of the X-ray powder diffraction pattern shown in Figure 2.
2.24 A substantially crystalline form of the compound of formula (13) having an X-ray powder diffraction pattern substantially as shown in Figure 2.

### Intermediate of Formula (14)

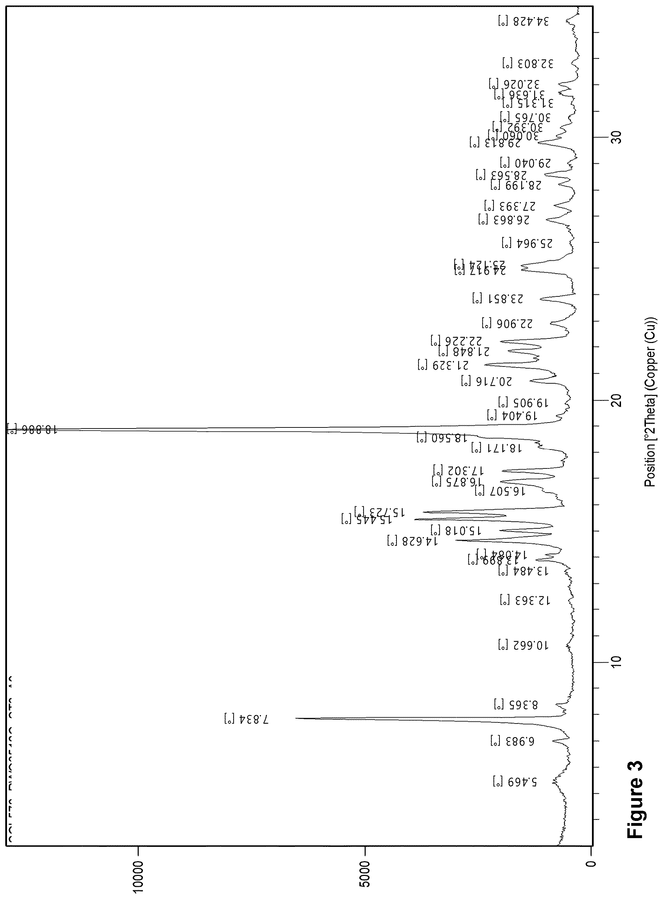

The XRPD diffractogram for a crystalline form of the intermediate of formula (14) is shown in Figure 3.

The X-ray diffraction pattern of the crystalline form of the intermediate of formula (14) exhibits peaks of greatest intensity at the diffraction angles (2θ) set out in Table A-3

| Table A-3 | |
|---|---|
| **Diffraction Angle (°)** | **Relative Intensity** |
| 7.8 ± 0.2 | 50 |
| 14.6 ± 0.2 | 19 |
| 15.4 ± 0.2 | 26 |
| 15.7 ± 0.2 | 24 |
| 18.9 ± 0.2 | 100 |

Accordingly, in further embodiments, the invention provides:
2.25 A substantially crystalline form of the compound of formula (14) having an X-ray powder diffraction pattern characterised by the presence of major peaks at the diffraction angles (2θ) 7.8° and/or 14.6° and/or 15.4° and/or 15.7° and/or 18.9° (±0.2°).
2.26 A substantially crystalline form of the compound of formula (14) having an X-ray powder diffraction pattern characterised by the presence of a major peak at the diffraction angle (2θ) 7.8° (±0.2°).
2.27 A substantially crystalline form of the compound of formula (14) having an X-ray powder diffraction pattern characterised by the presence of a major peak at the diffraction angle (2θ) 14.6° (±0.2°).
2.28 A substantially crystalline form of the compound of formula (14) having an X-ray powder diffraction pattern characterised by the presence of a major peak at the diffraction angle (2θ) 15.4° (±0.2°).
2.29 A substantially crystalline form of the compound of formula (14) having an X-ray powder diffraction pattern characterised by the presence of a major peak at the diffraction angle (2θ) 15.7° (±0.2°).
2.30 A substantially crystalline form of the compound of formula (14) having an X-ray powder diffraction pattern characterised by the presence of a major peak at the diffraction angle (2θ) 18.9° (±0.2°).
2.31 A substantially crystalline form of the compound of formula (14) having an X-ray powder diffraction pattern characterised by the presence of major peaks at two or more, e.g. three or more, or four or more, and in particular five diffraction angles (2θ) selected from 10.7°, 14.6°, 15.4°, 15.7° and 18.9° (±0.2°).
2.32 A substantially crystalline form of the compound of formula (14) which exhibits peaks at the diffraction angles set forth in Table A-3 provided above or Table 3 provided below which have a relative intensity of at least 15%.
2.33 A substantially crystalline form of the compound of formula (14) which exhibits peaks at the diffraction angles corresponding to those of the X-ray powder diffraction pattern shown in Figure 3.
2.34 A substantially crystalline form of the compound of formula (14) having an X-ray powder diffraction pattern substantially as shown in Figure 3.

Further aspects and embodiments of the invention will be apparent from the Examples provided below.

### BRIEF DESCRIPTION OF THE DRAWINGS

Figure 1 is a XRPD Pattern of a crystalline form of the intermediate of formula (12).
Figure 2 is a XRPD Pattern of a crystalline form of the intermediate of formula (13).
Figure 3 is a XRPD Pattern of a crystalline form of the intermediate of formula (14).
Figure 4 is a XRPD Pattern of a crystalline form of the intermediate of formula (15).
Figure 5 is a XRPD Pattern of a crystalline form of the intermediate of formula (17).
Figure 6 is a XRPD Pattern of a crystalline form of a benzenesulphonate salt of the intermediate of formula (18).
Figure 7 is a XRPD Pattern of a crystalline form of the free base of the intermediate of formula (18).

### EXAMPLES

The invention will now be illustrated, but not limited, by reference to the specific embodiments described in the following examples.

### Abbreviations

In the examples, the following abbreviations are used.
- AcOH: acetic acid
- aq: aqueous
- BSA: benzenesulfonic acid
- DAST: diethylaminosulfur trifluoride
- DCM: dichloromethane
- DIPEA: N,N-diisopropylethylamine
- DMSO: dimethylsulfoxide
- EtOAc: ethyl acetate
- EtOH: ethanol
- h: hour(s)
- HPLC: high performance liquid chromatography
- iPrOAc: isopropyl acetate
- iPrMgCl: isopropylmagnesium chloride
- KF: Karl Fisher
- LC: liquid chromatography
- LCMS: liquid chromatography-mass spectrometry
- MeCN: acetonitrile
- min: minute(s)
- MgSO₄: magnesium sulfate
- NMR: nuclear magnetic resonance
- RT: retention time
- THF: tetrahydrofuran

### Analytical Methods

### HPLC Method 1

HPLC analysis was carried out on an Agilent 1100 series HPLC system. The column used was an Aquity BEH Phenyl; 30 x 4.6mm, 1.7 µm particle size (Ex Waters, PN: 186004644). The flow rate was 2.0 mL/min. Mobile phase A was Water : Trifluoroacetic acid (100:0.03%) and mobile phase B was Acetonitrile : Trifluoroacetic acid (100:0.03%). Detection was by UV at 210 nm. The injection volume was 5 µL, column temperature 40 °C and the following gradient was used:

| Time | %A | %B |
|---|---|---|
| 0 | 95 | 5 |
| 5.2 | 5 | 95 |
| 5.7 | 5 | 95 |
| 5.8 | 95 | 5 |
| 6.2 | 95 | 5 |

### HPLC Method 2

HPLC analysis was carried out on an Agilent 1110 series HPLC system. The column used was an Aquity BEH Phenyl; 30 x 4.6mm, 1.7 µm particle size (Ex Waters, PN: 186004644). The flow rate was 2.0 mL/min. Mobile phase A was Water : Trifluoroacetic acid (100:0.03%) and mobile phase B was Acetonitrile : Trifluoroacetic acid (100:0.03%). Detection was by UV at 210 nm. The injection volume was 5 µL, column temperature 40 °C and the following gradient was used:

| **Time (min)** | **%A** | **%B** |
|---|---|---|
| 0 | 95 | 5 |
| 5 | 95 | 5 |
| 15 | 5 | 95 |
| 16 | 5 | 95 |
| 16.5 | 95 | 5 |
| 17 | 95 | 5 |

### HPLC Method 3

HPLC analysis was carried out on an Agilent 1100/1200 series liquid chromatograph. The column used was an XSelect Phenyl-Hexyl; 150 x 4.6mm, 2.5 µm particle size (Ex Waters, PN: 186006735). The flow rate was 1.0 mL/min. Mobile phase A was 10mM Ammonium Acetate (pH 5.8) and mobile phase B was Acetonitrile 100%. Detection was by UV at 302 nm. The injection volume was 5 µL and column temperature 50 °C and the following gradient was used:

| **Time (min)** | **%A** | **%B** |
|---|---|---|
| 0 | 95 | 5 |
| 20 | 5 | 95 |
| 24.5 | 5 | 95 |
| 25 | 95 | 5 |

### Proton-NMR

Structures of all intermediates were confirmed from their ¹H NMR spectra which were collected using a JEOL ECX 400MHz spectrometer equipped with an auto-sampler. The samples were dissolved in a suitable deuterated solvent for analysis. The data was acquired using Delta NMR Processing and Control Software version 4.3.

### X-Ray Powder Diffraction (XRPD)

X-Ray Powder Diffraction patterns were collected on a PANalytical diffractometer using Cu Kα radiation (45kV, 40mA), θ - θ goniometer, focusing mirror, divergence slit (1/2"), soller slits at both incident and divergent beam (4mm) and a PIXcel detector.

The software used for data collection was X'Pert Data Collector, version 2.2f and the data was presented using X'Pert Data Viewer, version 1.2d. XRPD patterns were acquired under ambient conditions via a transmission foil sample stage (polyimide - Kapton, 12.7µm thickness film) under ambient conditions using a PANalytical X'Pert PRO. The data collection range was 2.994 - 35°2θ with a continuous scan speed of 0.202004°s-1.

### Example 1

5-[[5-[4-(4-Fluoro-1-methyl-4-piperidyl)-2-methoxy-phenyl]-1H-pyrazol-3-yl]amino]pyrazine-2-carbonitrile was prepared as outlined in the reaction scheme shown below.

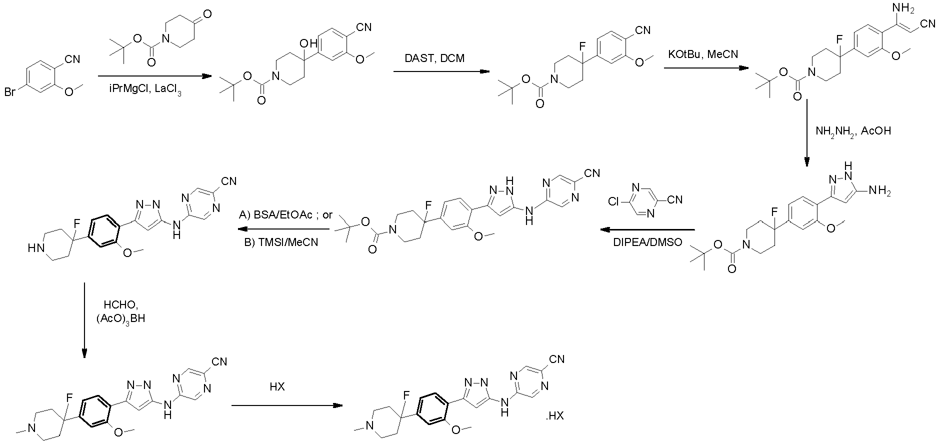

### Step 1: tert-Butyl 4-(4-cyano-3-methoxy-phenyl)-4-hydroxy-piperidine-1-carboxylate

To a 2 L vessel under nitrogen was charged 4-bromo-2-methoxybenzonitrile (125 g, 589 mmol) and anhydrous THF (375 mL). The slurry was cooled to 0°C. A 2M solution of iPrMgCl in THF (530 mL, 1.06 mol) was charged at 0-10°C over 20 min. The batch was stirred out at 0-5°C for 2 h. A 0.6M solution of LaCl₃.2LiCl in THF (196 mL, 118 mmol) was charged over 15 min at 0-5°C. After 30 min, a solution of N-Boc-4-piperidone (146.7 g, 736 mmol) in anhydrous THF (375 mL) was charged over 25 min at 0-15°C. After 30 min stirring, 10% AcOH (750 mL, 1.31 mol) was charged over 15 min at 0-30°C. The organic layer was separated off and concentrated to remove residual THF. The aqueous layer was extracted with TBME (750 mL) and combined with the concentrated organic layer. Water (250 mL) was charged, the batch stirred and the organic layer separated off and dried (MgSO₄). The batch was concentrated *in vacuo.* The crude material was dissolved in diisopropylether (500 mL) and transferred to a clean 2L vessel along with additional diisopropylether (125 mL). The batch was heated to 60°C and heptanes (500 mL) charged over 15 min at 55-60°C. The batch was cooled to 10°C over 5 h and then stirred overnight to give a slurry. Heptanes (375 mL) were charged, and the batch cooled to 0 °C for 30 min. The solids were filtered off and washed with an ice-cold mixture of diisopropylether (125 mL) and heptanes (125 mL). The material was dried at 40°C to afford the title compound as a white solid (126 g, 64% yield).

HPLC (Method 1) RT 2.97 min, 99.0%. ¹H NMR purity >95%.

¹H NMR (CDCl₃) δ 7.49 (d, *J* = 8.0 Hz, 1H), 7.16 (s, 1H), 7.04 (d, *J* = 6.4 Hz, 1H), 4.05 (br s, 2H), 3.93 (s, 3H), 3.89 (brs, 2H), 2.04 (s, 1H), 1.94 (brs, 2H), 1.67 (d, *J* = 12.8 Hz, 2H), 1.457 (s, 9H).

The XRPD Diffraction Pattern of the product is shown in Figure 1 and a list of XRPD peaks is shown in Table 1 below.

**Table 1**

| **Pos. [°2Th.]** | **Height [cts]** | **FWHM [°2Th.]** | **d-spacing [Å]** | **Rel. Int. [%]** |
|---|---|---|---|---|
| 5.6000 | 1735.69 | 0.0768 | 15.78189 | 21.76 |
| 8.4803 | 292.33 | 0.0768 | 10.42688 | 3.66 |
| 8.8402 | 201.55 | 0.0768 | 10.00328 | 2.53 |
| 10.5646 | 4320.08 | 0.0768 | 8.37400 | 54.16 |
| 11.3121 | 936.29 | 0.1023 | 7.82226 | 11.74 |
| 11.6533 | 594.49 | 0.0768 | 7.59402 | 7.45 |
| 12.3565 | 122.26 | 0.1023 | 7.16341 | 1.53 |
| 13.0852 | 3130.62 | 0.0768 | 6.76603 | 39.25 |
| 13.2823 | 7977.08 | 0.1023 | 6.66609 | 100.00 |
| 14.0532 | 2954.03 | 0.1023 | 6.30211 | 37.03 |
| 14.5131 | 2409.62 | 0.1279 | 6.10342 | 30.21 |
| 15.2683 | 1843.56 | 0.1279 | 5.80317 | 23.11 |
| 15.6991 | 525.46 | 0.1023 | 5.64489 | 6.59 |
| 16.6487 | 4864.57 | 0.1279 | 5.32502 | 60.98 |
| 17.0043 | 7918.32 | 0.1279 | 5.21443 | 99.26 |
| 17.4105 | 1874.34 | 0.1279 | 5.09370 | 23.50 |
| 17.7688 | 744.06 | 0.1279 | 4.99179 | 9.33 |
| 18.4513 | 203.57 | 0.1279 | 4.80864 | 2.55 |
| 18.9950 | 271.50 | 0.1023 | 4.67223 | 3.40 |
| 19.9165 | 4151.30 | 0.1279 | 4.45808 | 52.04 |
| 20.5065 | 1486.53 | 0.1023 | 4.33113 | 18.64 |
| 21.1127 | 3158.29 | 0.1279 | 4.20812 | 39.59 |
| 21.3170 | 1437.40 | 0.0768 | 4.16824 | 18.02 |
| 21.7063 | 653.99 | 0.1023 | 4.09436 | 8.20 |
| 22.0421 | 1189.78 | 0.1279 | 4.03274 | 14.92 |
| 22.4436 | 940.52 | 0.1535 | 3.96150 | 11.79 |
| 22.8199 | 488.42 | 0.1023 | 3.89701 | 6.12 |
| 23.1044 | 159.67 | 0.1535 | 3.84967 | 2.00 |
| 23.5132 | 264.26 | 0.1279 | 3.78366 | 3.31 |
| 24.4148 | 1857.74 | 0.1535 | 3.64593 | 23.29 |
| 24.6907 | 2508.89 | 0.1535 | 3.60581 | 31.45 |
| 25.0496 | 309.28 | 0.1791 | 3.55497 | 3.88 |
| 25.7063 | 1933.03 | 0.2303 | 3.46562 | 24.23 |
| 26.1593 | 391.11 | 0.1535 | 3.40662 | 4.90 |
| 26.4576 | 86.37 | 0.1023 | 3.36889 | 1.08 |
| 26.8516 | 160.52 | 0.1791 | 3.32034 | 2.01 |
| 27.2606 | 435.57 | 0.1791 | 3.27145 | 5.46 |
| 27.7590 | 318.75 | 0.1279 | 3.21383 | 4.00 |
| 28.4296 | 471.16 | 0.1535 | 3.13953 | 5.91 |
| 28.6924 | 469.86 | 0.1791 | 3.11138 | 5.89 |
| 29.3541 | 477.03 | 0.2814 | 3.04273 | 5.98 |
| 30.3127 | 416.40 | 0.1791 | 2.94865 | 5.22 |
| 30.9007 | 88.21 | 0.1535 | 2.89387 | 1.11 |
| 31.5317 | 736.32 | 0.2047 | 2.83738 | 9.23 |
| 32.1524 | 173.14 | 0.1023 | 2.78402 | 2.17 |
| 32.8573 | 113.25 | 0.1791 | 2.72588 | 1.42 |
| 33.3269 | 36.43 | 0.1535 | 2.68854 | 0.46 |
| 33.8229 | 506.29 | 0.2047 | 2.65024 | 6.35 |
| 34.5475 | 84.42 | 0.1535 | 2.59629 | 1.06 |

### Step 2: tert-Butyl 4-(4-cyano-3-methoxy-phenyl)-4-fluoro-piperidine-1-carboxylate

To a 2 L flask under nitrogen was charged DCM (1.2 L) and DAST (69.8 g, 133 mmol). The solution was cooled to -10°C. A solution of tert-butyl 4-(4-cyano-3-methoxyphenyl)-4-hydroxy-piperidine-1-carboxylate (120 g, 361 mmol) in DCM (0.24 L) was charged over 1 h at -10°C. After the addition was complete the reaction was warmed to 15-20°C for 1 h. To a 2nd vessel was charged potassium bicarbonate (240 g, 2.40 mol) and water (1 L). The batch was stirred at room temperature to give a solution. The reaction solution was transferred into the aq. potassium bicarbonate solution over 20 min (nitrogen transfer) at 15-25°C. DCM (50 mL) was used as a line rinse. The quenched batch was stirred for 15 min, and the pH checked (>7). The organic layer was separated off. The aqueous layer was extracted with DCM (240 mL), with water (150 mL) added to aid the separation. The organic layers were combined, dried (MgSO₄) and concentrated in vacuo. Diisopropylether (360 mL) was added to dissolve the crude material and concentrated in vacuo to give a white solid (114 g). To a portion of the crude material (100 g) was charged diisopropylether (400 mL) and heptanes (400 mL). The batch was heated to 70 °C to afford a solution, which was cooled to ambient temperature over 1 h and stirred overnight. The batch was filtered, and the solids washed with a mixture of diisopropylether (100 mL) and heptanes (100 mL) to afford 80 g. The material was purified further, diisopropylether (240 mL) and heptanes (240 mL) were added. The batch was heated to 70°C to afford a solution which was cooled to ambient temperature over 1 h. The batch was filtered, and the solids washed with a mixture of diisopropylether (80 mL) and heptanes (80 mL) to afford the title compound as a white solid (72.6 g, 69% yield). HPLC (method 2) RT 10.58 min, 99.2%. ¹H NMR purity >95%.

¹H NMR (CDCl₃) δ 7.54 (d, *J =* 8.8 Hz, 1H), 7.01 (s, 1H), 6.91 (d, *J =* 8.0 Hz, 1H), 4.13 (br s, 2H), 3.95 (s, 3H), 3.14 (brs, 2H), 2.00-1.91 (m, 4H), 1.28 (s, 9H).

The XRPD Diffraction Pattern of the product is shown in Figure 2 and a list of XRPD peaks is shown in Table 2 below.

**Table 2**

| **Pos. [°2Th.]** | **Height [cts]** | **FWHM [°2Th.]** | **d-spacing [Å]** | **Rel. Int. [%]** |
|---|---|---|---|---|
| 5.5759 | 250.24 | 0.3070 | 15.85005 | 4.90 |
| 7.5637 | 321.23 | 0.1023 | 11.68836 | 6.29 |
| 7.7929 | 345.44 | 0.1023 | 11.34503 | 6.76 |
| 8.5029 | 2545.21 | 0.1023 | 10.39922 | 49.80 |
| 8.8671 | 2621.51 | 0.1023 | 9.97295 | 51.30 |
| 9.8931 | 290.90 | 0.1023 | 8.94087 | 5.69 |
| 10.2574 | 791.90 | 0.0768 | 8.62410 | 15.50 |
| 10.7802 | 4447.72 | 0.1023 | 8.20702 | 87.03 |
| 11.2298 | 256.52 | 0.1023 | 7.87941 | 5.02 |
| 11.9615 | 1025.58 | 0.1023 | 7.39905 | 20.07 |
| 12.4050 | 1531.64 | 0.1023 | 7.13550 | 29.97 |
| 12.9069 | 2973.82 | 0.0768 | 6.85914 | 58.19 |
| 13.0954 | 1177.13 | 0.0512 | 6.76078 | 23.03 |
| 13.5236 | 3618.70 | 0.1023 | 6.54770 | 70.81 |
| 13.9781 | 4563.06 | 0.1023 | 6.33579 | 89.29 |
| 14.2608 | 2190.09 | 0.1023 | 6.21081 | 42.85 |
| 14.6423 | 474.92 | 0.0768 | 6.04984 | 9.29 |
| 14.8791 | 553.09 | 0.0768 | 5.95410 | 10.82 |
| 15.3807 | 1482.72 | 0.1023 | 5.76104 | 29.01 |
| 15.7382 | 4638.02 | 0.1279 | 5.63096 | 90.75 |
| 16.3321 | 1449.96 | 0.1791 | 5.42752 | 28.37 |
| 16.7161 | 2547.86 | 0.1023 | 5.30370 | 49.85 |
| 16.9480 | 990.51 | 0.0512 | 5.23164 | 19.38 |
| 17.2303 | 1839.29 | 0.1279 | 5.14654 | 35.99 |
| 17.7874 | 2089.46 | 0.1023 | 4.98661 | 40.88 |
| 18.1798 | 668.68 | 0.1279 | 4.87985 | 13.08 |
| 18.6918 | 2219.86 | 0.1279 | 4.74732 | 43.44 |
| 18.8618 | 1893.05 | 0.1279 | 4.70490 | 37.04 |
| 19.2793 | 497.37 | 0.0768 | 4.60395 | 9.73 |
| 19.4832 | 937.67 | 0.1279 | 4.55623 | 18.35 |
| 19.9611 | 751.31 | 0.1023 | 4.44820 | 14.70 |
| 20.2790 | 900.98 | 0.1535 | 4.37920 | 17.63 |
| 20.7647 | 1357.93 | 0.1279 | 4.27785 | 26.57 |
| 21.0645 | 1929.94 | 0.0768 | 4.21764 | 37.76 |
| 21.7471 | 5110.63 | 0.1279 | 4.08677 | 100.00 |
| 22.1444 | 252.94 | 0.1535 | 4.01434 | 4.95 |
| 22.6313 | 1101.76 | 0.1535 | 3.92906 | 21.56 |
| 23.1518 | 1724.31 | 0.2047 | 3.84189 | 33.74 |
| 23.8098 | 608.11 | 0.1535 | 3.73720 | 11.90 |
| 24.3171 | 813.83 | 0.1535 | 3.66036 | 15.92 |
| 24.5245 | 646.68 | 0.1023 | 3.62987 | 12.65 |
| 25.1723 | 668.59 | 0.1535 | 3.53791 | 13.08 |
| 25.5338 | 301.43 | 0.1279 | 3.48863 | 5.90 |
| 25.8660 | 765.04 | 0.1279 | 3.44459 | 14.97 |
| 26.4572 | 570.66 | 0.1023 | 3.36894 | 11.17 |
| 26.7806 | 622.82 | 0.1279 | 3.32898 | 12.19 |
| 27.3338 | 304.36 | 0.1279 | 3.26286 | 5.96 |
| 27.6556 | 424.70 | 0.1791 | 3.22562 | 8.31 |
| 28.0073 | 120.65 | 0.1279 | 3.18590 | 2.36 |
| 28.6144 | 358.98 | 0.1791 | 3.11968 | 7.02 |
| 29.0738 | 202.73 | 0.1535 | 3.07142 | 3.97 |
| 29.4243 | 290.93 | 0.1023 | 3.03563 | 5.69 |
| 30.0826 | 345.67 | 0.1535 | 2.97068 | 6.76 |
| 30.9128 | 96.16 | 0.2047 | 2.89277 | 1.88 |
| 31.8509 | 152.62 | 0.1535 | 2.80968 | 2.99 |
| 32.7872 | 63.21 | 0.2047 | 2.73155 | 1.24 |
| 33.2960 | 75.62 | 0.2047 | 2.69097 | 1.48 |
| 34.2242 | 231.14 | 0.2047 | 2.62007 | 4.52 |

### Step 3: tert-butyl 4-[4-[(Z)-1-amino-2-cyano-vinyl]-3-methoxy-phenyl]-4-fluoro-piperidine-1-carboxylate

To a 2L vessel was charged tert-butyl 4-(4-cyano-3-methoxy-phenyl)-4-fluoro-piperidine-1-carboxylate (60.2 g, 180 mmol) and anhydrous THF (180.6 mL). The batch was stirred at 15-25°C to achieve a solution. Anhydrous MeCN (18.7 mL, 360 mmol) was charged. A 1M solution of potassium tert-butoxide in THF (540 mL, 540 mmol) was charged over 10 min at 15-25°C followed by a line rinse (THF 60 mL). The batch was then warmed to 40°C for 4 h. The batch was cooled to 15-25°C and water (16.3 mL, 900 mmol) charged. After 10 min, the batch was concentrated to ~1/4 volume on the rotavapor. The crude material was slurried in water (903 mL) for 30 min at 15-25°C and then filtered. The solids were washed with water (300 mL) and dried at 40°C to afford the title compound as a tan solid (65.0 g, 96% yield). HPLC (method 2): RT 10.46 min, 94.4% (plus 1.4% β- ketonitrile). ¹H NMR purity >95%.
¹H NMR (CDCl₃) δ 7.39 (d, *J* = 8.0 Hz, 1H), 7.02 (s, 1H), 6.91 (d, *J* = 7.2 Hz, 1H), 5.31 (brs, 2H), 4.19 (s, 1H), 4.15 (brs, 2H), 3.92 (s, 3H), 3.17 (brs, 2H), 1.98-1.92 (m, 4H), 1.50 (s, 9H)

The XRPD Diffraction Pattern of the product is shown in Figure 3 and a list of XRPD peaks is shown in Table 3 below.

**Table 3**

| **Pos. [°2Th.]** | **Height [cts]** | **FWHM [°2Th.]** | **d-spacing [Å]** | **Rel. Int. [%]** |
|---|---|---|---|---|
| 5.4689 | 240.23 | 0.3582 | 16.15972 | 1.94 |
| 6.9829 | 350.51 | 0.1023 | 12.65909 | 2.83 |
| 7.8336 | 6225.23 | 0.0768 | 11.28620 | 50.34 |
| 8.3646 | 316.68 | 0.1535 | 10.57091 | 2.56 |
| 10.6621 | 112.95 | 0.3070 | 8.29763 | 0.91 |
| 12.3627 | 78.59 | 0.2047 | 7.15979 | 0.64 |
| 13.4836 | 60.04 | 0.1791 | 6.56700 | 0.49 |
| 13.8994 | 643.56 | 0.0768 | 6.37149 | 5.20 |
| 14.0839 | 397.10 | 0.0512 | 6.28842 | 3.21 |
| 14.6277 | 2289.66 | 0.1023 | 6.05586 | 18.52 |
| 15.0181 | 1309.61 | 0.1023 | 5.89932 | 10.59 |
| 15.4453 | 3272.62 | 0.1023 | 5.73708 | 26.47 |
| 15.7226 | 3017.13 | 0.1535 | 5.63651 | 24.40 |
| 16.5070 | 378.31 | 0.1023 | 5.37040 | 3.06 |
| 16.8754 | 1343.83 | 0.1535 | 5.25397 | 10.87 |
| 17.3024 | 1331.68 | 0.1279 | 5.12528 | 10.77 |
| 18.1709 | 526.77 | 0.1023 | 4.88221 | 4.26 |
| 18.5597 | 1711.79 | 0.0512 | 4.78081 | 13.84 |
| 18.8857 | 12365.81 | 0.1279 | 4.69902 | 100.00 |
| 19.4039 | 218.88 | 0.0768 | 4.57467 | 1.77 |
| 19.9053 | 47.78 | 0.1279 | 4.46055 | 0.39 |
| 20.7157 | 785.19 | 0.1023 | 4.28786 | 6.35 |
| 21.3288 | 1704.68 | 0.1279 | 4.16597 | 13.79 |
| 21.8478 | 1251.73 | 0.1279 | 4.06816 | 10.12 |
| 22.2264 | 1458.26 | 0.1535 | 3.99972 | 11.79 |
| 22.9060 | 453.68 | 0.1535 | 3.88256 | 3.67 |
| 23.8508 | 728.77 | 0.1279 | 3.73086 | 5.89 |
| 24.9169 | 1056.31 | 0.1023 | 3.57360 | 8.54 |
| 25.1239 | 1076.77 | 0.1279 | 3.54462 | 8.71 |
| 25.9638 | 51.11 | 0.1535 | 3.43183 | 0.41 |
| 26.8630 | 528.50 | 0.1535 | 3.31896 | 4.27 |
| 27.3926 | 395.22 | 0.1279 | 3.25599 | 3.20 |
| 28.1993 | 309.51 | 0.1279 | 3.16465 | 2.50 |
| 28.5628 | 638.11 | 0.1279 | 3.12519 | 5.16 |
| 29.0396 | 145.63 | 0.1535 | 3.07496 | 1.18 |
| 29.8128 | 764.20 | 0.2047 | 2.99695 | 6.18 |
| 30.0601 | 346.53 | 0.1023 | 2.97285 | 2.80 |
| 30.3923 | 252.10 | 0.1535 | 2.94111 | 2.04 |
| 30.7651 | 93.59 | 0.1535 | 2.90632 | 0.76 |
| 31.3153 | 74.69 | 0.1535 | 2.85650 | 0.60 |
| 31.6360 | 282.60 | 0.1535 | 2.82827 | 2.29 |
| 32.0261 | 405.16 | 0.1279 | 2.79471 | 3.28 |
| 32.8034 | 143.11 | 0.1279 | 2.73024 | 1.16 |
| 34.4285 | 200.93 | 0.2047 | 2.60500 | 1.62 |

### Step 4: tert-butyl 4-[4-(5-amino-1H-pyrazol-3-yl)-3-methoxy-phenyl]-4-fluoro-piperidine-1-carboxylate

To tert-butyl 4-[4-[(Z)-1-amino-2-cyano-vinyl]-3-methoxy-phenyl]-4-fluoro-piperidine-1-carboxylate (61.4 g, 163.6 mmol) was charged EtOH (246 mL) and the batch stirred at 15-25°C. Hydrazine monohydrate (9.6 mL, 196 mmol) was charged followed by glacial acetic acid (11.2 mL, 196 mmol). The batch was warmed to 75°C for 2 h. The batch was cooled to 15-25°C, and water (614 mL) charged. After 30 min stirring, the solids were filtered off and washed with water (2x246 mL). The material was dried at 50°C to afford the title compound an off-white solid (58.6 g, 91.7% yield). HPLC (method 1): 97.7% (1.6% amide). KF water measurement: 1.3%.

To reduce levels of the amide side product a portion of the material (49.4g) was slurried at 50°C with diisopropyl ether (250 mL) for 30 min and then isolated at 15-25°C. The solids were washed with diisopropyl ether (50 mL) and oven dried to afford 47.7g stage 4. HPLC (method): 0.8% amide. A portion of the material (41.5g) was dissolved in DCM (415 mL) at 15-25°C and washed with 1M K₂CO₃ (2x100 mL). The organic layer was dried (MgSO4), concentrated and oven dried at 50°C to afford the title compound (39.7 g, 58% yield) as a pale-yellow solid. HPLC (method 3): RT 12.94 min, 96.4%. ¹H NMR purity >95%.

¹H NMR (CDCl₃) δ 10.35 (s, 1H), 7.54 (d, *J* = 7.6 Hz, 1H), 7.04 (s, 1H), 6.93 (d, *J =* 8.0 Hz, 1H), 5.99 (s, 1H), 4.16 (brs, 2H), 3.98 (s, 3H), 3.16 (brs, 2H), 3.16 (brs, 2H), 2.06-1.94 (m, 4H), 1.48 (s, 9H).

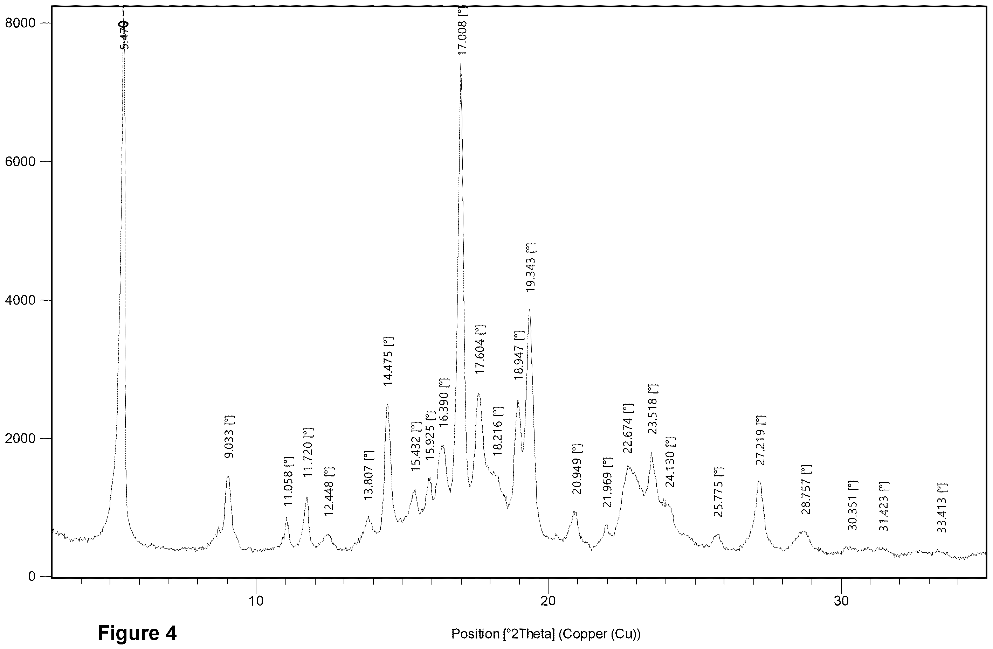

The XRPD Diffraction Pattern of the product is shown in Figure 4 and a list of XRPD peaks is shown in Table 4 below.

**Table 4**

| **Pos. [°2Th.]** | **Height [cts]** | **FWHM [°2Th.]** | **d-spacing [Å]** | **Rel. Int. [%]** |
|---|---|---|---|---|
| 5.4701 | 7934.94 | 0.1023 | 16.15636 | 100.00 |
| 9.0334 | 1038.47 | 0.1535 | 9.78967 | 13.09 |
| 11.0578 | 329.27 | 0.1023 | 8.00157 | 4.15 |
| 11.7201 | 725.22 | 0.1279 | 7.55088 | 9.14 |
| 12.4477 | 195.58 | 0.2047 | 7.11111 | 2.46 |
| 13.8066 | 261.35 | 0.1023 | 6.41411 | 3.29 |
| 14.4749 | 1757.29 | 0.1791 | 6.11945 | 22.15 |
| 15.4324 | 355.29 | 0.1791 | 5.74185 | 4.48 |
| 15.9245 | 437.99 | 0.1535 | 5.56549 | 5.52 |
| 16.3898 | 862.30 | 0.2558 | 5.40854 | 10.87 |
| 17.0079 | 6332.70 | 0.1791 | 5.21334 | 79.81 |
| 17.6039 | 1629.07 | 0.1535 | 5.03817 | 20.53 |
| 18.2161 | 517.84 | 0.1535 | 4.87020 | 6.53 |
| 18.9472 | 1746.24 | 0.1791 | 4.68389 | 22.01 |
| 19.3431 | 3115.50 | 0.2303 | 4.58891 | 39.26 |
| 20.9486 | 457.60 | 0.1791 | 4.24072 | 5.77 |
| 21.9689 | 245.25 | 0.1023 | 4.04602 | 3.09 |
| 22.6739 | 946.42 | 0.3070 | 3.92178 | 11.93 |
| 23.5183 | 1201.83 | 0.1535 | 3.78285 | 15.15 |
| 24.1304 | 458.22 | 0.2558 | 3.68826 | 5.77 |
| 25.7751 | 195.93 | 0.2558 | 3.45652 | 2.47 |
| 27.2189 | 933.74 | 0.2047 | 3.27637 | 11.77 |
| 28.7572 | 263.63 | 0.4093 | 3.10451 | 3.32 |
| 30.3511 | 41.55 | 0.6140 | 2.94501 | 0.52 |
| 31.4234 | 54.23 | 0.3070 | 2.84692 | 0.68 |
| 33.4134 | 66.59 | 0.4093 | 2.68178 | 0.84 |

### Step 5: tert-butyl 4-[4-[5-[(5-cyanopyrazin-2-yl)amino]-1H-pyrazol-3-yl]-3-methoxyphenyl]-4-fluoro-piperidine-1-carboxylate

To a 500 mL vessel under nitrogen was charged tert-butyl 4-[4-(5-amino-1H-pyrazol-3-yl)-3-methoxy-phenyl]-4-fluoro-piperidine-1-carboxylate (32.4 g, 83 mmol) followed by 5-chloro-2-cyanopyrazine (12.34 g, 91.3 mmol) and anhydrous DMSO (64.8 mL). DIPEA (18.1 ml, 103.8 mmol) was charged, and the batch warmed to 50°C for 20 h. The batch was cooled to ambient temperature and iPrOAc (162 mL) charged. The resulting solution was poured into water (324 mL) at 15-30°C, and the batch stirred for 1 h. The batch was filtered and washed with water (324 mL) and iPrOAc (162 mL). The solids were oven dried at 50°C to afford the title compound as a tan solid (39.6g, 94% yield). HPLC (method 3): RT 15.57 min, 97.6%. ¹H NMR purity >95%.

¹H NMR (DMSO-d6) δ 12.65 (s, 1H), 10.76 (s, 1H), 8.66 (s, 1H), 8.51 (brs, 1H), 7.67 (d, *J =* 8.0 Hz, 1H), 7.14 (s, 1H), 7.09 (d, *J* = 7.2 Hz, 1H), 4.01 (brs, 2H), 3.93 (s, 3H), 3.05 (brs, 2H), 2.13-1.88 (m, 4H), 1.42 (s, 9H).

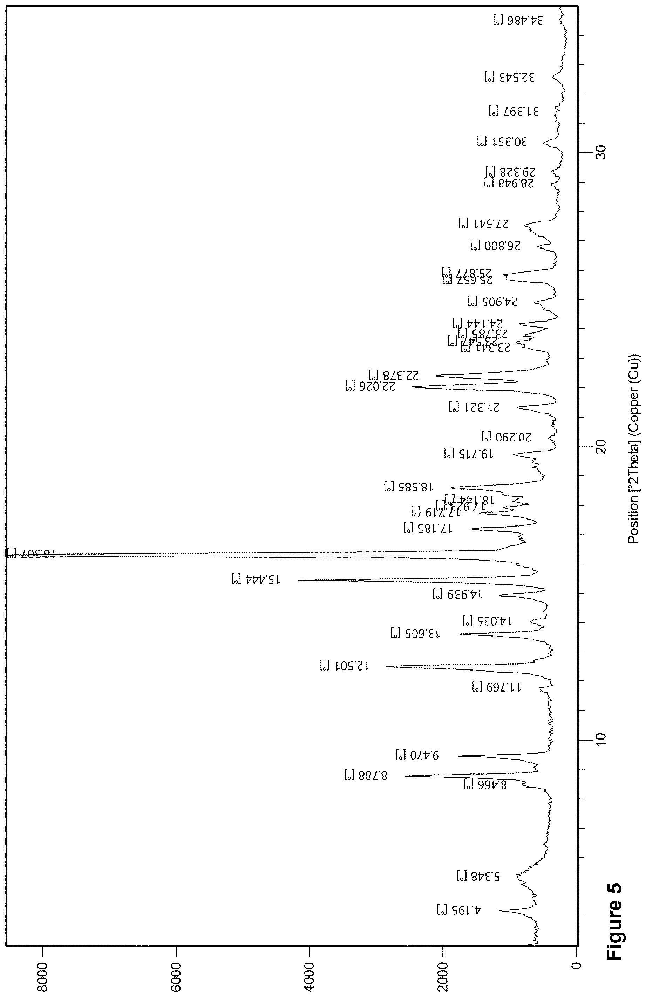

The XRPD Diffraction Pattern of the product is shown in Figure 5 and a list of XRPD peaks is shown in Table 5 below.

**Table 5**

| **Pos. [°2Th.]** | **Height [cts]** | **FWHM [°2Th.]** | **d-spacing [Å]** | **Rel. Int. [%]** |
|---|---|---|---|---|
| 4.1954 | 569.49 | 0.0768 | 21.06200 | 7.12 |
| 5.3479 | 308.77 | 0.2558 | 16.52527 | 3.86 |
| 8.4656 | 363.88 | 0.1279 | 10.44499 | 4.55 |
| 8.7877 | 2144.83 | 0.1023 | 10.06290 | 26.83 |
| 9.4696 | 1383.59 | 0.1023 | 9.33971 | 17.31 |
| 11.7687 | 161.36 | 0.1279 | 7.51981 | 2.02 |
| 12.5013 | 2436.64 | 0.1023 | 7.08073 | 30.48 |
| 13.6052 | 1343.68 | 0.1279 | 6.50862 | 16.81 |
| 14.0353 | 243.89 | 0.1279 | 6.31012 | 3.05 |
| 14.9385 | 642.13 | 0.1279 | 5.93054 | 8.03 |
| 15.4436 | 3595.84 | 0.1279 | 5.73771 | 44.98 |
| 16.3066 | 7993.52 | 0.1279 | 5.43594 | 100.00 |
| 17.1845 | 911.03 | 0.1279 | 5.16015 | 11.40 |
| 17.7188 | 818.95 | 0.1023 | 5.00576 | 10.25 |
| 17.9227 | 471.10 | 0.0768 | 4.94926 | 5.89 |
| 18.1445 | 351.66 | 0.0768 | 4.88926 | 4.40 |
| 18.5853 | 1311.37 | 0.1279 | 4.77428 | 16.41 |
| 19.7149 | 578.53 | 0.1279 | 4.50319 | 7.24 |
| 20.2896 | 95.06 | 0.1535 | 4.37693 | 1.19 |
| 21.3211 | 567.64 | 0.1535 | 4.16744 | 7.10 |
| 22.0264 | 2078.15 | 0.1279 | 4.03558 | 26.00 |
| 22.3775 | 1697.27 | 0.1535 | 3.97304 | 21.23 |
| 23.3408 | 330.06 | 0.1279 | 3.81121 | 4.13 |
| 23.5470 | 528.13 | 0.1023 | 3.77831 | 6.61 |
| 23.7850 | 396.22 | 0.1023 | 3.74103 | 4.96 |
| 24.1442 | 509.60 | 0.1279 | 3.68618 | 6.38 |
| 24.9050 | 295.74 | 0.1023 | 3.57528 | 3.70 |
| 25.6568 | 660.72 | 0.1279 | 3.47219 | 8.27 |
| 25.8774 | 678.62 | 0.1535 | 3.44308 | 8.49 |
| 26.8002 | 260.78 | 0.1535 | 3.32659 | 3.26 |
| 27.5412 | 451.11 | 0.1535 | 3.23876 | 5.64 |
| 28.9481 | 108.84 | 0.1535 | 3.08447 | 1.36 |
| 29.3280 | 100.05 | 0.1535 | 3.04538 | 1.25 |
| 30.3511 | 235.36 | 0.1279 | 2.94501 | 2.94 |
| 31.3973 | 62.73 | 0.5117 | 2.84923 | 0.78 |
| 32.5428 | 164.39 | 0.1279 | 2.75150 | 2.06 |
| 34.4863 | 17.53 | 0.2558 | 2.60076 | 0.22 |

### Step 6A: 5-[[3-[4-(4-fluoro-4-piperidyl)-2-methoxy-phenyl]-1H-pyrazol-5-yllaminolpyrazine-2-carbonitrile

To a flask under nitrogen was charged tert-butyl 4-[4-[5-[(5-cyanopyrazin-2-yl)amino]-1H-pyrazol-3-yl]-3-methoxy-phenyl]-4-fluoro-piperidine-1-carboxylate (1 g, 2.03 mmol) and 1,4-dioxane (15 mL). The batch was heated to 80-85°C for 1 h then cooled to ambient and the solvent was removed *in vacuo.* To the crude material was charged EtOAc (30 mL) and the batch heated to 60°C. Benzenesulfonic acid (801 mg, 5.08 mmol) was charged, and the slurry heated at 60°C for 26 h. The batch was cooled to ambient and filtered. The solids were washed with EtOAc (10 mL) and oven dried at 50°C to afford the title compound as its besylate salt as a light brown solid (1.15 g, 84%). HPLC (method 3): RT 9.09 min, 97.9%. ¹H NMR: 41% BSA, 0.8% EtOAc, 58% title compound.

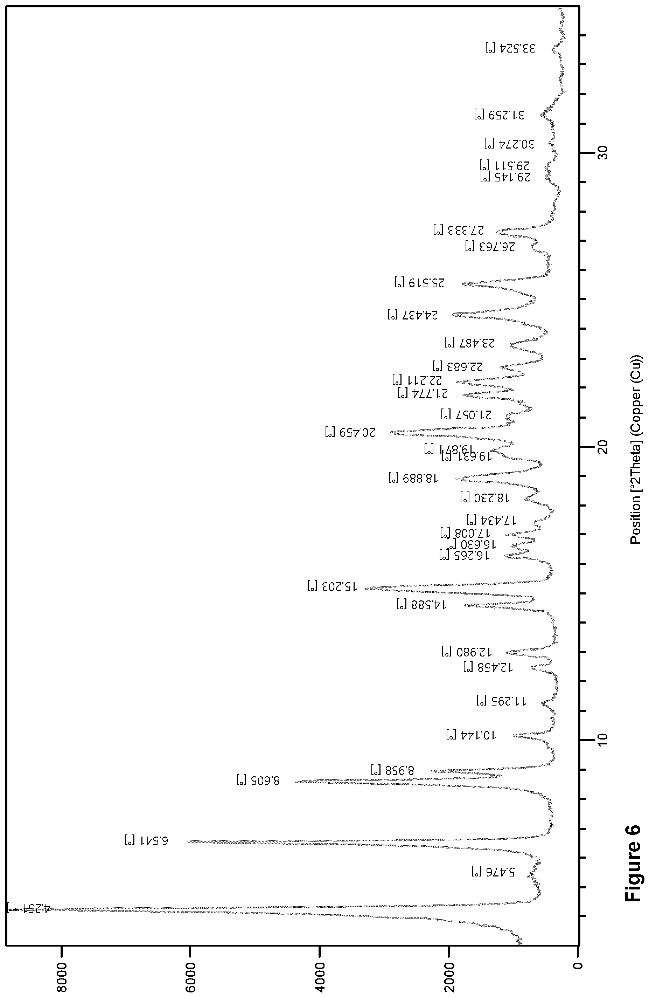

The XRPD Diffraction Pattern of the product is shown in Figure 6 and a list of XRPD peaks is shown in Table 6 below.

**Table 6**

| **Pos. [°2Th.]** | **Height [cts]** | **FWHM [°2Th.]** | **d-spacing [Å]** | **Rel. Int. [%]** |
|---|---|---|---|---|
| 4.2512 | 8202.52 | 0.1279 | 20.78537 | 100.00 |
| 5.4758 | 110.94 | 0.4093 | 16.13946 | 1.35 |
| 6.5407 | 5574.71 | 0.1279 | 13.51398 | 67.96 |
| 8.6054 | 3916.84 | 0.1279 | 10.27559 | 47.75 |
| 8.9584 | 1846.09 | 0.1279 | 9.87150 | 22.51 |
| 10.1438 | 613.00 | 0.1279 | 8.72042 | 7.47 |
| 11.2947 | 167.75 | 0.1791 | 7.83431 | 2.05 |
| 12.4583 | 383.33 | 0.1279 | 7.10507 | 4.67 |
| 12.9796 | 700.07 | 0.1791 | 6.82084 | 8.53 |
| 14.5882 | 1324.20 | 0.1279 | 6.07217 | 16.14 |
| 15.2029 | 2717.18 | 0.2814 | 5.82799 | 33.13 |
| 16.2645 | 656.33 | 0.1279 | 5.44991 | 8.00 |
| 16.6296 | 534.95 | 0.1535 | 5.33109 | 6.52 |
| 17.0081 | 631.25 | 0.1279 | 5.21329 | 7.70 |
| 17.4344 | 246.19 | 0.1535 | 5.08675 | 3.00 |
| 18.2303 | 299.90 | 0.1791 | 4.86644 | 3.66 |
| 18.8886 | 1329.06 | 0.2558 | 4.69829 | 16.20 |
| 19.6313 | 441.99 | 0.1535 | 4.52220 | 5.39 |
| 19.8707 | 686.74 | 0.1023 | 4.46825 | 8.37 |
| 20.4588 | 2148.08 | 0.2303 | 4.34112 | 26.19 |
| 21.0567 | 337.15 | 0.2047 | 4.21919 | 4.11 |
| 21.7739 | 1025.60 | 0.1791 | 4.08180 | 12.50 |
| 22.2106 | 1157.29 | 0.2047 | 4.00252 | 14.11 |
| 22.6825 | 592.56 | 0.2047 | 3.92031 | 7.22 |
| 23.4872 | 498.04 | 0.2303 | 3.78779 | 6.07 |
| 24.4366 | 1367.64 | 0.2303 | 3.64273 | 16.67 |
| 25.5187 | 1290.15 | 0.2047 | 3.49066 | 15.73 |
| 26.7626 | 252.40 | 0.2047 | 3.33119 | 3.08 |
| 27.3332 | 764.66 | 0.2558 | 3.26293 | 9.32 |
| 29.1449 | 145.36 | 0.2558 | 3.06409 | 1.77 |
| 29.5113 | 139.30 | 0.2558 | 3.02688 | 1.70 |
| 30.2736 | 39.41 | 0.3070 | 2.95237 | 0.48 |
| 31.2593 | 222.46 | 0.2047 | 2.86148 | 2.71 |
| 33.5239 | 148.11 | 0.1791 | 2.67319 | 1.81 |

### Step 6B: 5-[[3-[4-(4-fluoro-4-piperidyl)-2-methoxy-phenyl]-1H-pyrazol-5-yllaminolpyrazine-2-carbonitrile

As an alternative to Step 6A above, 5-[[3-[4-(4-fluoro-4-piperidyl)-2-methoxy-phenyl]-1H-pyrazol-5-yl]amino]pyrazine-2-carbonitrile was also prepared according to the following method.

To tert-butyl 4-[4-[5-[(5-cyanopyrazin-2-yl)amino]-1H-pyrazol-3-yl]-3-methoxy-phenyl]-4-fluoro-piperidine-1-carboxylate (1.0 g, 2.03 mmo) was added MeCN (10 mL) followed by iodotrimethylsilane (577 µl, 4.06 mmol) at 0-20°C. After 30 min 10% aqueous potassium carbonate (5 mL, 3.62 mmol) was charged (off-gassing observed) and the batch stirred for 30 min. The solids were then filtered off and washed with a mixture of MeCN (2 mL) and water (2 mL). The solids were oven dried at 50°C to afford the title compound as a light brown solid (766 mg, 96%). HPLC (method 3): RT 9.09 mun, 98.8%. ¹H NMR purity >95%.

¹H NMR (DMSO-d6) δ 12.65 (s, 1H), 10.50 (s, 1H), 8.66 (s, 1H), 8.51 (brs,1H), 7.67 (d, *J =* 8.4 Hz, 1H), 7.10 (s, 1H), 7.05 (d, *J =* 8.0 Hz, 1H), 3.92 (s, 3H), 3.48 (brs, 1H), 2.93-2.83 (m, 4H), 2.08-1.82 (m, 4H).

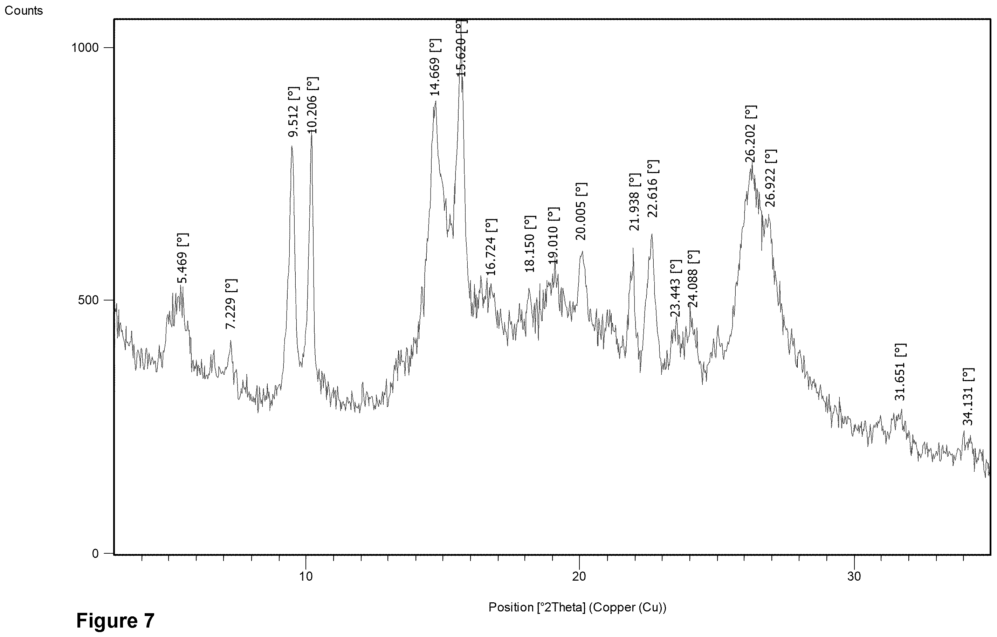

The XRPD Diffraction Pattern of the product is shown in Figure 7 and a list of XRPD peaks is shown in Table 7 below.

| **Pos. [°2Th.]** | **Height [cts]** | **FWHM [°2Th.]** | **d-spacing [Å]** | **Rel. Int. [%]** |
|---|---|---|---|---|
| 5.4686 | 133.02 | 0.3070 | 16.16070 | 23.89 |
| 7.2285 | 87.91 | 0.2047 | 12.22954 | 15.78 |
| 9.5118 | 475.92 | 0.1535 | 9.29834 | 85.46 |
| 10.2059 | 482.92 | 0.1279 | 8.66747 | 86.71 |
| 14.6692 | 481.13 | 0.2047 | 6.03884 | 86.39 |
| 15.6196 | 556.90 | 0.1279 | 5.67344 | 100.00 |
| 16.7239 | 82.50 | 0.5117 | 5.30122 | 14.81 |
| 18.1495 | 75.78 | 0.1535 | 4.88791 | 13.61 |
| 19.0095 | 84.49 | 0.4093 | 4.66868 | 15.17 |
| 20.0050 | 137.18 | 0.2303 | 4.43855 | 24.63 |
| 21.9383 | 205.92 | 0.1023 | 4.05158 | 36.98 |
| 22.6163 | 255.58 | 0.1279 | 3.93164 | 45.89 |
| 23.4429 | 70.03 | 0.2558 | 3.79484 | 12.58 |
| 24.0880 | 91.80 | 0.3070 | 3.69466 | 16.48 |
| 26.2023 | 373.13 | 0.6140 | 3.40113 | 67.00 |
| 26.9223 | 294.89 | 0.3070 | 3.31178 | 52.95 |
| 31.6514 | 54.38 | 0.4093 | 2.82693 | 9.76 |
| 34.1306 | 47.75 | 0.4093 | 2.62705 | 8.57 |

### Step 7: 5-[[3-[4-(4-fluoro-1-methyl-4-piperidyl)-2-methoxy-phenyl]-1H-pyrazol-5-yl]amino]pyrazine-2-carbonitrile

To 5-[[3-[4-(4-fluoro-4-piperidyl)-2-methoxy-phenyl]-1H-pyrazol-5-yl]amino]pyrazine-2-carbonitrile (60.79 g active, 155 mmol) was charged EtOH (608 mL). The batch was stirred at RT and 37% formalin (22.5 mL, 278 mmol) charged. Sodium triacetoxyborohydride (58.95 g, 278 mmol) was charged in four portions over 30 min at 15-25°C. After 2 h, an 8.5% ammonium hydroxide solution (608 mL) was charged over 20 min at <25°C. The batch was cooled to 0-5°C and filtered, washing the cake with water (2 x 304 mL). The wet material was returned to vessel with water (608 mL) and slurried at RT for 30 min. The batch was filtered and washed with water (304 mL). The solids were oven dried at 50°C to afford the title compound (51.4 g, 82% active yield (100% minus water and ethanol)).

HPLC (Method 3) RT 10.20 min, 97.0%. 1H NMR purity >95%.

1H NMR (DMSO-d6) δ 12.64 (s, 1H), 10.74 (s, 1H), 8.66 (s, 1H), 8.51 (brs,1H), 7.66 (d, *J =* 8.0 Hz, 1H), 7.12 (s, 1H), 7.07 (d, *J* = 8.8 Hz, 1H), 3.92 (s, 3H), 2.72-2.49 (m, 2H), 2.22-2.09 (m, 4H), 2.12 (s, 3H), 1.93-1.86 (m, 2H).

### Equivalents

The foregoing examples are presented for the purpose of illustrating the invention and should not be construed as imposing any limitation on the scope of the invention. It will readily be apparent that numerous modifications and alterations may be made to the specific embodiments of the invention described above and illustrated in the examples without departing from the scope of the claims. All such modifications and alterations are intended to be embraced by this invention.

## Claims

1. A process for the preparation of a compound of formula (15): 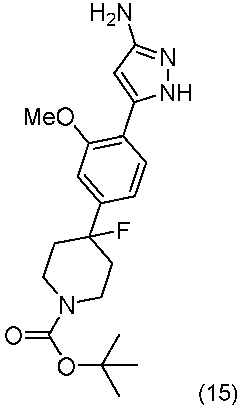 which process comprises reacting a compound of the formula (14): 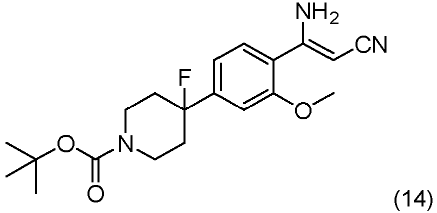 with hydrazine (NH₂NH₂).

2. A process according to claim 1 wherein the reaction is carried out in a polar, protic solvent, optionally wherein the polar, protic solvent is a C₁₋₄ alcohol, such as ethanol (EtOH).

3. A process according to claim 1 or claim 2 wherein the reaction is carried out in the presence of glacial acetic acid.

4. A process according to any one of claims 1 to 3 wherein the reaction is carried out at a temperature of 70 °C to 80 °C, for example at a temperature of 75 °C.

5. A process according to any one of claims 1 to 4 wherein the reaction is carried out for a time period of 4 hours or less, for example for a time period of from 1 hour to 3 hours.

6. A process for the preparation of 5-[[5-[4-(4-fluoro-1-methyl-4-piperidyl)-2-methoxy-phenyl]-1H-pyrazol-3-yl]amino]pyrazine-2-carbonitrile, which process comprises:
i) a process according to claim 1; and
ii) interconverting a product obtained from step i) into 5-[[5-[4-(4-fluoro-1-methyl-4-piperidyl)-2-methoxy-phenyl]-1H-pyrazol-3-yl]amino]pyrazine-2-carbonitrile; and
iii) optionally forming a pharmaceutically acceptable salt of 5-[[5-[4-(4-fluoro-1-methyl-4-piperidyl)-2-methoxy-phenyl]-1H-pyrazol-3-yl]amino]pyrazine-2-carbonitrile.

7. A process for the preparation of 5-[[5-[4-(4-fluoro-1-methyl-4-piperidyl)-2-methoxy-phenyl]-1H-pyrazol-3-yl]amino]pyrazine-2-carbonitrile, which process comprises:
i) a process according to claim 1;
ii) interconverting a product obtained from step i) to a compound of formula (18): 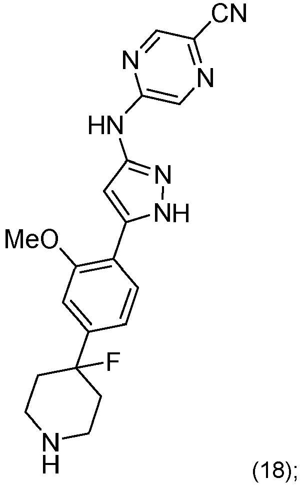
iii) interconverting a product obtained from step ii) into 5-[[5-[4-(4-fluoro-1-methyl-4-piperidyl)-2-methoxy-phenyl]-1H-pyrazol-3-yl]amino]pyrazine-2-carbonitrile; and
iv) optionally forming a pharmaceutically acceptable salt of 5-[[5-[4-(4-fluoro-1-methyl-4-piperidyl)-2-methoxy-phenyl]-1H-pyrazol-3-yl]amino]pyrazine-2-carbonitrile.

8. A process according to claim 7 wherein the product obtained from step ii) is interconverted into 5-[[5-[4-(4-fluoro-1-methyl-4-piperidyl)-2-methoxy-phenyl]-1H-pyrazol-3-yl]amino]pyrazine-2-carbonitrile by reacting the compound of formula (18) with a methylating agent (such as HCHO in the presence of a reducing agent such as (AcO₃)BH).

9. A process for the preparation of 5-[[5-[4-(4-fluoro-1-methyl-4-piperidyl)-2-methoxy-phenyl]-1H-pyrazol-3-yl]amino]pyrazine-2-carbonitrile, which process comprises:
i) a process for the preparation of a compound of formula (15) according to claim 1;
ii) reacting the compound of formula (15) with a compound of formula (16): 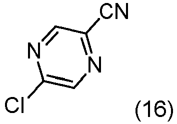 to form a compound of formula (17): 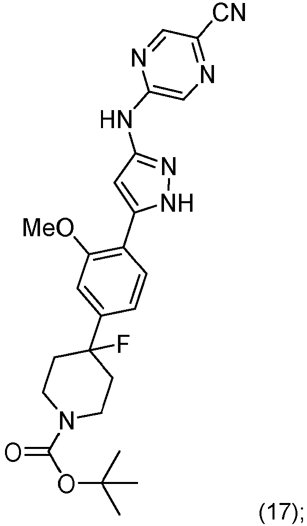
ii) reacting the compound of formula (17) with an alkylsilyl halide or a sulphonic acid to form a compound of formula (18): 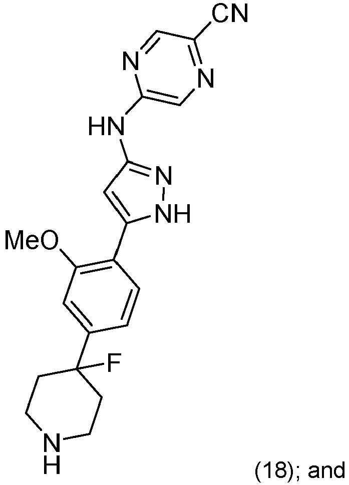
ii) interconverting the compound of formula (18) obtained from step i) into 5-[[5-[4-(4-fluoro-1-methyl-4-piperidyl)-2-methoxy-phenyl]-1H-pyrazol-3-yl]amino]pyrazine-2-carbonitrile, for example by reacting the compound of formula (18) with a methylating agent (such as HCHO in the presence of a reducing agentsuch as (AcO₃)BH); and
iii) optionally forming a pharmaceutically acceptable salt of 5-[[5-[4-(4-fluoro-1-methyl-4-piperidyl)-2-methoxy-phenyl]-1H-pyrazol-3-yl]amino]pyrazine-2-carbonitrile.

10. A process according to claim 9, wherein the process further comprises preparing the compound of formula (14) by reacting a compound of formula (13): 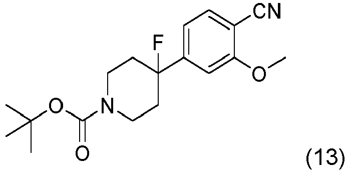 with acetonitrile (CH₃CN) in the presence of a base.

11. A process according to claim 10, wherein the process further comprises preparing the compound of formula (13) by reacting a compound of formula (12): 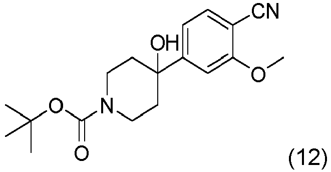 with a deoxyfluorination reagent.

12. A process according to claim 11, wherein the process further comprises preparing the compound of formula (12) by reacting a compound of formula (10): 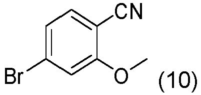 with a compound of formula (11): 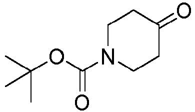 in the presence of a metallating agent, such as a Grignard reagent.

13. A compound selected from:
(a) a compound of the formula (14): 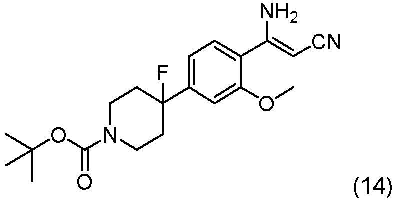
(b) a compound of the formula (13): 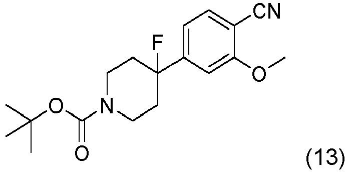
(c) a compound of the formula (12):

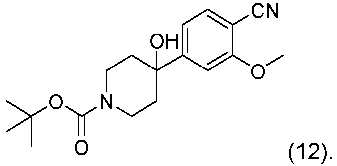

14. A compound according to claim 13 in a crystalline form.

15. A compound according to claim 14 wherein the compound is of the formula (14) and has an Xray powder diffraction pattern **characterised by** the presence of major peaks at two or more, e.g. three or more, or four or more, and in particular five diffraction angles (2θ) selected from 7.8°, 14.6°, 15.4°, 15.7° and 18.9° (±0.2°).

## Patentansprüche

1. Prozess zur Herstellung einer Verbindung der Formel (15): 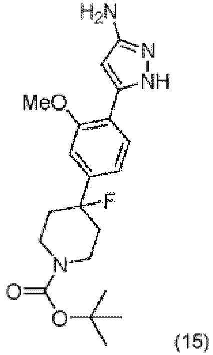 wobei der Prozess Reagierenlassen einer Verbindung der Formel (14) umfasst: 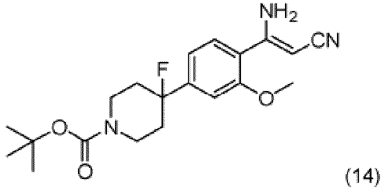 mit Hydrazin (NH₂NH₂).

2. Prozess nach Anspruch 1, wobei die Reaktion in einem polaren, protischen Lösungsmittel ausgeführt wird, wobei das polare, protische Lösungsmittel optional ein C₁₋₄-Alkohol ist, wie etwa Ethanol (EtOH).

3. Prozess nach Anspruch 1 oder Anspruch 2, wobei die Reaktion in Gegenwart von Eisessig ausgeführt wird.

4. Prozess nach einem der Ansprüche 1 bis 3, wobei die Reaktion bei einer Temperatur von 70 °C bis 80 °C, zum Beispiel bei einer Temperatur von 75 °C, ausgeführt wird.

5. Prozess nach einem der Ansprüche 1 bis 4, wobei die Reaktion über einen Zeitraum von 4 Stunden oder weniger, zum Beispiel über einen Zeitraum von 1 Stunde bis 3 Stunden, ausgeführt wird.

6. Prozess zur Herstellung von 5-[[5-[4-(4-Fluor-1-methyl-4-piperidyl)-2-methoxy-phenyl]-1H-pyrazol-3-yl]amino]pyrazin-2-carbonitril, wobei der Prozess Folgendes umfasst:
i) einen Prozess nach Anspruch 1; und
ii) Umwandeln eines aus Schritt i) erhaltenen Produkts in 5-[[5-[4-(4-Fluor-1-methyl-4-piperidyl)-2-methoxy-phenyl]-1H-pyrazol-3-yl]amino]pyrazin-2-carbonitril; und
iii) optional Bilden eines pharmazeutisch unbedenklichen Salzes von 5-[[5-[4-(4-Fluor-1-methyl-4-piperidyl)-2-methoxy-phenyl]-1H-pyrazol-3-yl]amino]pyrazin-2-carbonitril.

7. Prozess zur Herstellung von 5-[[5-[4-(4-Fluor-1-methyl-4-piperidyl)-2-methoxy-phenyl]-1H-pyrazol-3-yl]amino]pyrazin-2-carbonitril, wobei der Prozess Folgendes umfasst:
i) einen Prozess nach Anspruch 1;
ii) Umwandeln eines aus Schritt i) erhaltenen Produkts in eine Verbindung der Formel (18): 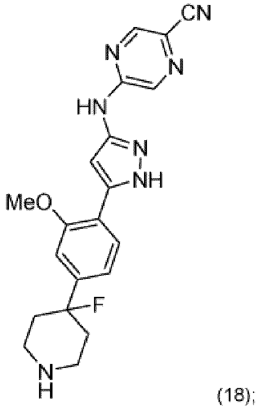
iii) Umwandeln eines aus Schritt ii) erhaltenen Produkts in 5-[[5-[4-(4-Fluor-1-methyl-4-piperidyl)-2-methoxy-phenyl]-1H-pyrazol-3-yl]amino]pyrazin-2-carbonitril; und
iv) optional Bilden eines pharmazeutisch unbedenklichen Salzes von 5-[[5-[4-(4-Fluor-1-methyl-4-piperidyl)-2-methoxy-phenyl]-1H-pyrazol-3-yl]amino]pyrazin-2-carbonitril.

8. Prozess nach Anspruch 7, wobei das in Schritt ii) erhaltene Produkt durch Reagierenlassen der Verbindung der Formel (18) mit einem Methylierungsmittel (wie etwa HCHO in Gegenwart eines Reduktionsmittels, wie etwa (AcO₃)BH), in 5-[[5-[4-(4-Fluor-1-methyl-4-piperidyl)-2-methoxy-phenyl]-1H-pyrazol-3-yl]amino]pyrazin-2-carbonitril umgewandelt wird.

9. Prozess zur Herstellung von 5-[[5-[4-(4-Fluor-1-methyl-4-piperidyl)-2-methoxy-phenyl]-1H-pyrazol-3-yl]amino]pyrazin-2-carbonitril, wobei der Prozess Folgendes umfasst:
i) einen Prozess zur Herstellung einer Verbindung der Formel (15) nach Anspruch 1;
ii) Reagierenlassen der Verbindung der Formel (15) mit einer Verbindung der Formel (16): 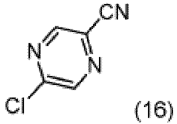 zum Bilden einer Verbindung der Formel (17): 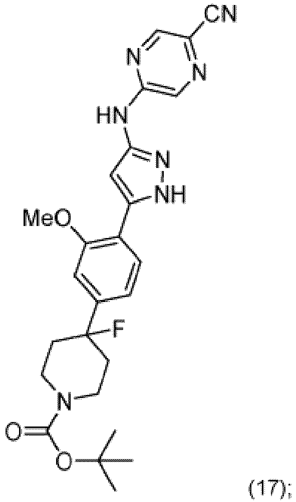
ii) Reagierenlassen der Verbindung der Formel (17) mit einem Alkylsilylhalogenid oder einer Sulfonsäure zum Bilden einer Verbindung der Formel (18): 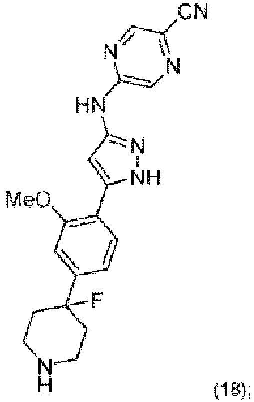 und
ii) Umwandeln der Verbindung der Formel (18), die aus Schritt i) erhalten wurde, in 5-[[5-[4-(4-Fluor-1-methyl-4-piperidyl)-2-methoxy-phenyl]-1H-pyrazol-3-yl]amino]pyrazin-2-carbonitril, zum Beispiel durch Reagierenlassen der Verbindung der Formel (18) mit einem Methylierungsmittel (wie etwa HCHO in Gegenwart eines Reduktionsmittels, wie etwa (AcO₃)BH); und
iii) optional Bilden eines pharmazeutisch unbedenklichen Salzes von 5-[[5-[4-(4-Fluor-1-methyl-4-piperidyl)-2-methoxy-phenyl]-1H-pyrazol-3-yl]amino]pyrazin-2-carbonitril.

10. Prozess nach Anspruch 9, wobei der Prozess ferner Herstellen der Verbindung der Formel (14) durch Reagierenlassen einer Verbindung der Formel (13): 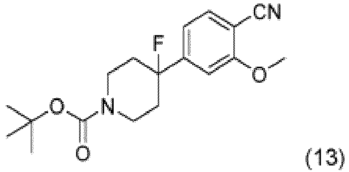 mit Acetonitril (CH₃CN) in Gegenwart einer Base umfasst.

11. Prozess nach Anspruch 10, wobei der Prozess ferner Herstellen der Verbindung der Formel (13) durch Reagierenlassen einer Verbindung der Formel (12): 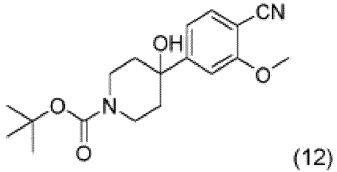 mit einem Desoxyfluorierungsreagenz umfasst.

12. Prozess nach Anspruch 11, wobei der Prozess ferner Herstellen der Verbindung der Formel (12) durch Reagierenlassen einer Verbindung der Formel (10): 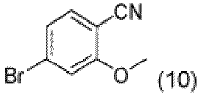 mit einer Verbindung der Formel (11): 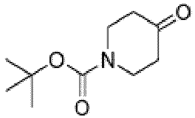 in Gegenwart eines Metallierungsmittels, wie etwa eines Grignard-Reagenz, umfasst.

13. Verbindung, ausgewählt aus:
(a) einer Verbindung der Formel (14): 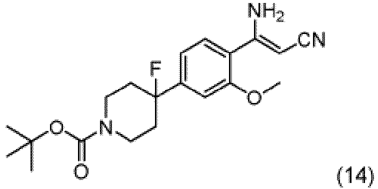
(b) einer Verbindung der Formel (13): 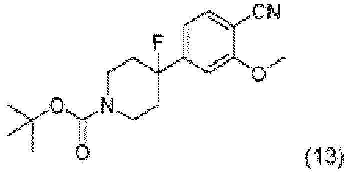
(c) einer Verbindung der Formel (12):

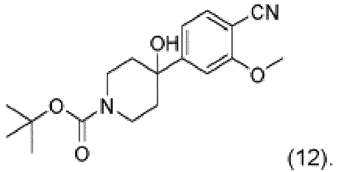

14. Verbindung nach Anspruch 13 in kristalliner Form.

15. Verbindung nach Anspruch 14, wobei die Verbindung der Formel (14) entspricht und ein Röntgenpulverbeugungsmuster aufweist, das durch die Gegenwart von Hauptspitzen bei zwei oder mehr, z. B. drei oder mehr oder vier oder mehr und insbesondere fünf Beugungswinkeln (20) gekennzeichnet ist, die aus 7,8°, 14,6°, 15,4°, 15,7° und 18,9° (±0,2°) ausgewählt sind.

## Revendications

1. Processus permettant la préparation d'un composé de formule (15) : 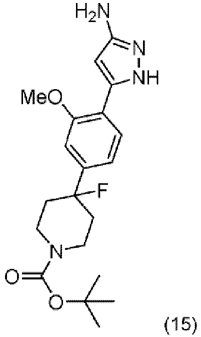 lequel processus comprend la mise en réaction d'un composé de la formule (14) : 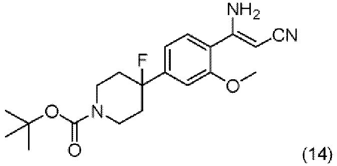 avec l'hydrazine (NH₂NH₂).

2. Processus selon la revendication 1, dans lequel la réaction est réalisée dans un solvant polaire protique, éventuellement dans lequel le solvant polaire protique est un alcool en C₁₋₄, tel que l'éthanol (EtOH).

3. Processus selon la revendication 1 ou la revendication 2, dans lequel la réaction est réalisée en présence d'acide acétique glacial.

4. Processus selon l'une quelconque des revendications 1 à 3, dans lequel la réaction est réalisée à une température de 70 °C à 80 °C, par exemple à une température de 75 °C.

5. Processus selon l'une quelconque des revendications 1 à 4, dans lequel la réaction est réalisée pendant une période de temps de 4 heures ou moins, par exemple pendant une période de temps de 1 heure à 3 heures.

6. Processus permettant la préparation de 5-[[5-[4-(4-fluoro-1-méthyl-4-pipéridyl)-2-méthoxy-phényl]-1H-pyrazol-3-yl]amino]pyrazine-2-carbonitrile, lequel processus comprend :
i) un processus selon la revendication 1 ; et
ii) l'interconversion d'un produit obtenu à l'étape i) en 5-[[5-[4-(4-fluoro-1-méthyl-4-pipéridyl)-2-méthoxy-phényl]-1H-pyrazol-3-yl]amino]pyrazine-2-carbonitrile ; et
iii) la formation éventuelle d'un sel pharmaceutiquement acceptable de 5-[[5-[4-(4-fluoro-1-méthyl-4-pipéridyl)-2-méthoxy-phényl]-1H-pyrazol-3-yl]amino]pyrazine-2-carbonitrile.

7. Processus permettant la préparation de 5-[[5-[4-(4-fluoro-1-méthyl-4-pipéridyl)-2-méthoxy-phényl]-1H-pyrazol-3-yl]amino]pyrazine-2-carbonitrile, lequel processus comprend :
i) un processus selon la revendication 1 ;
ii) l'interconversion d'un produit obtenu à l'étape i) en un composé de formule (18) : 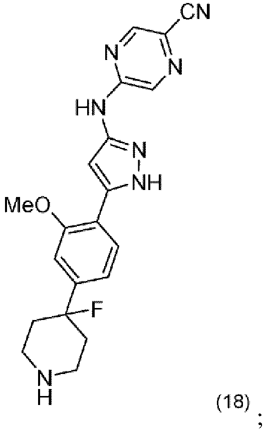
iii) l'interconversion d'un produit obtenu à l'étape ii) en 5-[[5-[4-(4-fluoro-1-méthyl-4-pipéridyl)-2-méthoxy-phényl]-1H-pyrazol-3-yl]amino]pyrazine-2-carbonitrile ; et
iv) la formation éventuelle d'un sel pharmaceutiquement acceptable de 5-[[5-[4-(4-fluoro-1-méthyl-4-pipéridyl)-2-méthoxy-phényl]-1H-pyrazol-3-yl]amino]pyrazine-2-carbonitrile.

8. Processus selon la revendication 7, dans lequel le produit obtenu à l'étape ii) est interconverti en 5-[[5-[4-(4-fluoro-1-méthyl-4-pipéridyl)-2-méthoxy-phényl]-1H-pyrazol-3-yl]amino]pyrazine-2-carbonitrile en faisant réagir le composé de formule (18) avec un agent de méthylation (tel que HCHO en présence d'un agent réducteur tel que (AcO₃)BH).

9. Processus permettant la préparation de 5-[[5-[4-(4-fluoro-1-méthyl-4-pipéridyl)-2-méthoxy-phényl]-1H-pyrazol-3-yl]amino]pyrazine-2-carbonitrile, lequel processus comprend :
i) un processus permettant la préparation d'un composé de formule (15) selon la revendication 1 ;
ii) la mise en réaction du composé de formule (15) avec un composé de formule (16) : 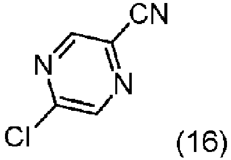 pour former un composé de formule (17) : 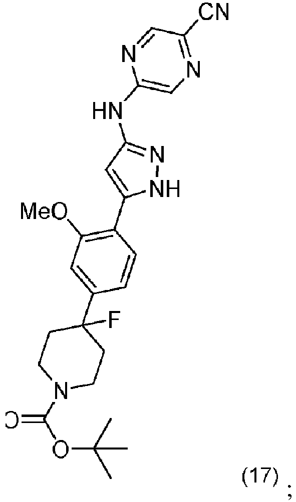
ii) la mise en réaction du composé de formule (17) avec un halogénure d'alkylsilyle ou un acide sulfonique pour former un composé de formule (18) : 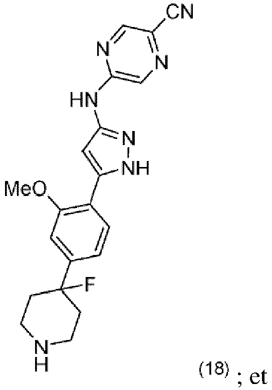
ii) l'interconversion du composé de formule (18) obtenu à l'étape i) en 5-[[5-[4-(4-fluoro-1-méthyl-4-pipéridyl)-2-méthoxy-phényl]-1H-pyrazol-3-yl]amino]pyrazine-2-carbonitrile, par exemple en faisant réagir le composé de formule (18) avec un agent de méthylation (tel que HCHO en présence d'un agent réducteur tel que (AcO₃)BH) ; et
iii) la formation éventuelle d'un sel pharmaceutiquement acceptable de 5-[[5-[4-(4-fluoro-1-méthyl-4-pipéridyl)-2-méthoxy-phényl]-1H-pyrazol-3-yl]amino]pyrazine-2-carbonitrile.

10. Processus selon la revendication 9, dans lequel le processus comprend en outre la préparation du composé de formule (14) en faisant réagir un composé de formule (13) : 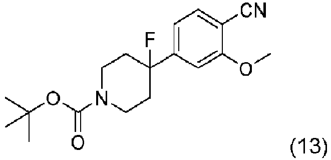 avec l'acétonitrile (CH₃CN) en présence d'une base.

11. Processus selon la revendication 10, dans lequel le processus comprend en outre la préparation du composé de formule (13) en faisant réagir un composé de formule (12) : 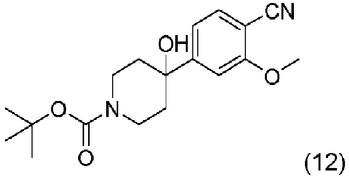 avec un réactif de désoxyfluoration.

12. Processus selon la revendication 11, dans lequel le processus comprend en outre la préparation du composé de formule (12) en faisant réagir un composé de formule (10) : 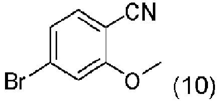 avec un composé de formule (11) : 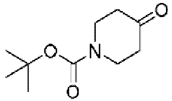 en présence d'un agent métallant, tel qu'un réactif de Grignard.

13. Composé choisi parmi :
(a) un composé de la formule (14) : 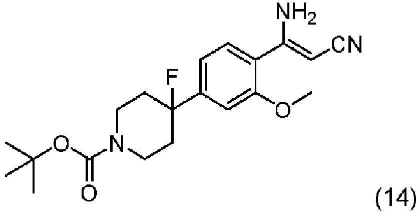
(b) un composé de la formule (13) : 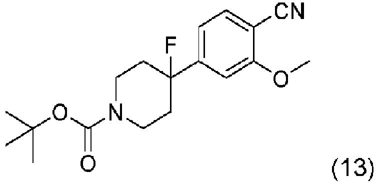
(c) un composé de la formule (12) :

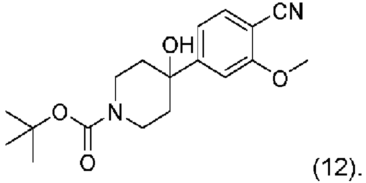

14. Composé selon la revendication 13 sous une forme cristalline.

15. Composé selon la revendication 14, dans lequel le composé répond à la formule (14) et comporte un diagramme de diffraction des rayons X sur poudre **caractérisé par** la présence de pics majeurs à deux angles de diffraction (2θ) ou plus, par exemple trois ou plus, ou quatre ou plus, et en particulier cinq, choisis parmi 7,8°, 14,6°, 15,4°, 15,7° et 18,9° (±0,2°).
